# EUROPEAN PATENT SPECIFICATION

(11) **EP 0 422 134 B1**
(45) Date of publication and mention of the grant of the patent: **12.06.1996**
(21) Application number: 90900592.8
(22) Date of filing: 07.12.1989
(51) Int. Cl.: C07D 471/00, C07D 491/056, C07D 513/02, A61K 31/40, A61K 31/415, A61K 31/42, A61K 31/425, A61K 31/435, A61K 31/44, A61K 31/47, A61K 31/475

(54) **5-HT SELECTIVE AGENTS**
5-HT-SELEKTIVE MITTEL
AGENTS SELECTIFS DE 5-HT

(30) Priority: 15.12.1988 US 285134
(43) Date of publication of application: 17.04.1991
(73) Proprietor: ABBOTT LABORATORIES, Abbott Park, IL 60064-2204 (US)
(72) Inventor: DeBERNARDIS, John, F., Lindenhurst, IL 60046 (US); MEYER, Michael, D., Lindenhurst, IL 60046 (US); SIPPY, Kevin, B., Lindenhurst, IL 60046 (US)
(74) Representative: Modiano, Guido, Dr.-Ing.
(86) International application number: US8905512
(87) International publication number: WO9006927

(56) References cited:
- EP-A- 9 566 6A2
- EP-A-17 009 3A1
- GB-A- 1 401 287
- NL-A- 7 216 762
- US-A- 4 341 786
- US-A- 4 612 316
- US-A- 4 618 683
- US-A- 4 662 405
- US-A- 4 740 602
- US-A- 4 842 852
- No further relevant documents have been disclosed.

## Description

### Background of the Invention

This invention relates to compounds and pharmaceutical compositions useful for the treatment anxiety, depression and hypertension. More particularly, this invention concerns certain novel 4-alkoxy-1,2,3,3a,8,8a-hexahydroindeno [1,2-c]pyrrole and 5-alkoxy-2,3,4,4a, 9,9a-hexahydro-1H-indeno[2,1-c]pyridine compounds useful in the treatment of anxiety, depression, and hypertension by virtue of their high affinity and selectivity for the 5-HT_{1A} (5-hydroxytryptamine) subtype of serotonin receptors.

The serotonergic system exerts a complex and as yet not fully understood control over cardiovascular and CNS function. Multiple subtypes of the serotonin receptor have been described; and specific functions have been ascribed to certain of these subtypes which would indicate that pharmacological intervention would produce beneficial therapeutic results.

In particular, the 5-HT_{1A} subtype of the serotonin receptor has been shown to exert significant control over the cardiovascular function, and may be involved as well in the etiology of anxiety and depression. Activation of centrally located 5-HT_{1A} receptors has been shown to have an inhibitory effect on sympathetic outflow. Selective agonists of this receptor site have been shown to be efficacious in the treatment of hypertension in experimental animals. Although the precise mechanism of this antihypertensive effect has not been unequivocally determined, the currently available evidence suggests that this response to 5-HT_{1A} selective agonists is mediated by decreased total peripheral resistance rather than decreased cardiac output. Therefore, modulation of central 5-HT_{1A} receptors represents a novel and potentially useful method for the control of hypertension not found in any currently available drug.

The use of 5-HT_{1A} selective agents in the treatment of anxiety has now become a clinically established principle. Again, the precise mechanisms by which these agents exert their anxiolytic effects have not been determined. Modulation of the serotonergic nervous system has become an equally well established principle for the treatment of depression, and current evidence has suggested that 5-HT_{lA} selective agents may have therapeutic efficacy. Serotonergic abnormalities have been suggested for a variety of additional disease states such as, but not limited to, migraine, schizophrenia,dementia, eating disorders, sexual disorders, and nausea. Serotonic receptor subtype selective agents may find utility in the treatment of such disorders.

United States Patent 4,618,683 discloses a class of tetrahydro-6,7-dimethoxy-1 H-benz[e]isoindoline compounds which interact specifically with various adrenergic receptors and, as a result, are useful sedative agents or agents useful in the treatment of hypertension.

EP-A-0 170 093 discloses indeno[1,2-c]pyrroles which interact specifically with various adrenergic receptors and are useful in the treatment of hypertension.

### Summary of the Invention

It has now been determined that the compounds of the present invention, as herein defined, demonstrate the ability to interact specifically with serotonin receptors and are thus useful as therapeutic agents in the treatment of hypertension, anxiety, and depression.

This invention relates to certain novel 4-alkoxy-1,2,3,3a,8,8a-hexahydroindeno[1,2-c]pyrrole and 5-alkoxy-2,3,4,4a,9,9a-hexahydro-1 H-indeno[2,1-c]pyridine compounds and to pharmaceutical compositions comprising these compounds.

The compounds of this invention are represented by the formula 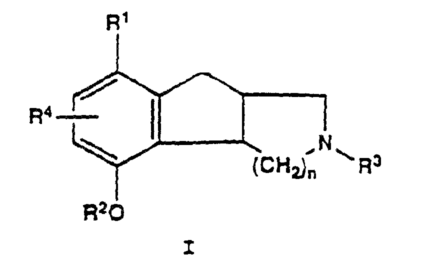 wherein
R¹ is hydrogen or an electron withdrawing group;
R² is hydrogen, lower alkyl, or arylalkyl; n is an integer having a value of from 1 to 3;
R⁴ is a member selected from the group consisting of hydrogen, halogen, lower alkyl, lower alkoxy, and arylalkyl; or
R²0 and R⁴ taken together form an alkylenedioxy bridge;
R³ is a member selected from the group consisting of arylamidoalkenylene, arylamidoalkylene, aryl(lower alkyl) amidoalkenylene, aryl (lower alkyl) amidoalkylene, benzoalkylenedioxyalkylene,
   a) a group of the formula: 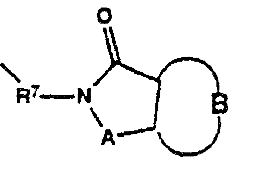
      wherein B is benzo, cyclohexyl, or a bicyclo ring;
      A is CO, SC, or S0₂, and
      R⁷ is a bivalent aliphatic hydrocarbon;
   b) a group of the formula: 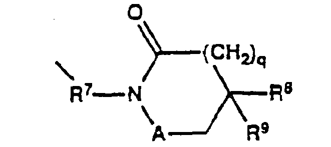 wherein q is an integer of from 0 to 3, R⁸ and R⁹ are hydrogen or lower alkyl, or R⁸ and R⁹ taken together form a ring of from 5 to 7 members and A and R⁷ are as defined above; and
   c) a group of the formula: 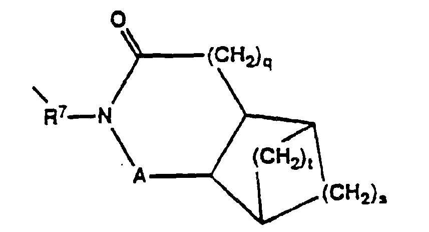 wherein s and t are independent integers of from 1 to 3, and q, A, and R⁷ are as defined above; and the pharmaceutically acceptable salts thereof.

### Detailed description of the Invention

The present invention provides novel compounds which demonstrate a high affinity and selectivity for 5-HT_{1A} subtype of serotonin receptors. This selectivity allows for the usefulness of the compounds for treating hypertension, anxiety, and depression.

The serotonergic nervous system plays a significant role in the control of various disease states including, but not limited to, hypertension, anxiety, and depression. Selective central activation of the 5-HT_{iA} receptor sub-population results in decreased sympathetic outflow, which can cause loweing of blood pressure, total peripheral resistance, and heart rate. Blockade, or partial blockade of this receptor sub-population results in alteration of serotonergic neuronal activity which is beneficial in the treatment of anxiety and depression.

The compounds of this invention are represented by the formula: 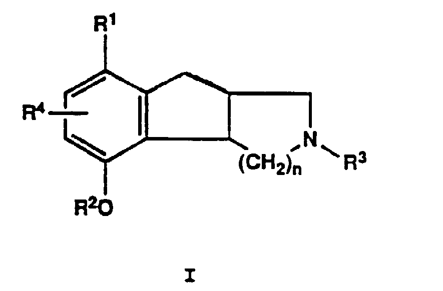
wherein R¹ is hydrogen or an electron withdrawing group;
R² is hydrogen, lower alkyl, or arylalkyl;
n is an integer having a value of from 1 to 3;
R⁴ is a member selected from the group consisting of hydrogen, halogen, lower alkyl, lower alkoxy, and arylalkyl; or R²0 and R⁴ taken together form an alkylenedioxy bridge;
R³ is a member selected from the group consisting of arylamidoalkenylene, arylamidoalkylene, aryl(lower alkyl)-amidoalkenylene, aryl(lower alkyl) amidoalkylene, benzoalkylenedioxyalkylene,
   a) a group of the formula: 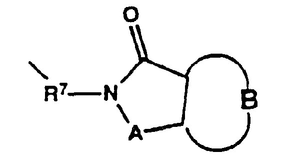
      wherein B is benzo, cyclohexyl, or a bicyclo ring;
      A is CO, SO, or S0₂, and
   b) a group of the formula: 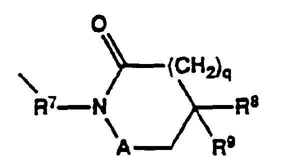 wherein q is an integer of from 0 to 3, R⁸ and R⁹ are hydrogen or lower alkyl,or R⁸ and R⁹ taken together form a ring of from 5 to 7 members and A and R⁷ are as defined above; and
   c) a group of the formula: 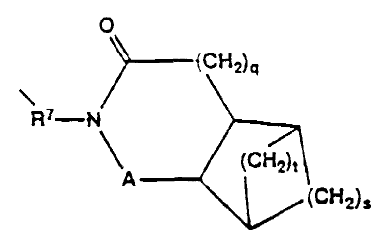 wherein s and t are independent integers of from 1 to 3, and q, A, and R⁷ are as defined above; and the pharmaceutically acceptable salts thereof.

This invention is also directed to pharmaceutical compositions and the use of compounds for manufacturing medicaments for treating anxiety, depression, hypertension, and related disorders.

Preferred compounds of the present invention are those wherein n is 1, represented by the formula: 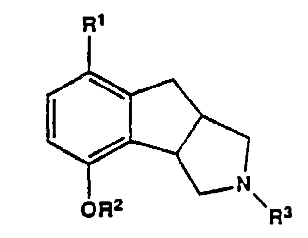 and compounds wherein h is 2, which are represented by the formula: 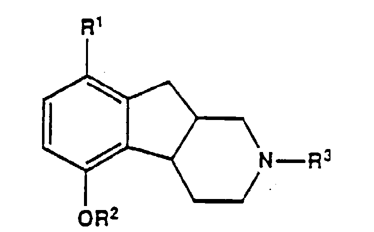 where R³ is selected from:

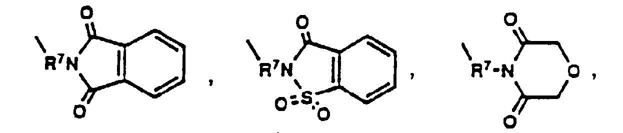

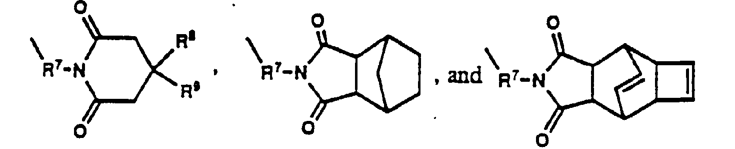

Ring systems in the R³ group including the benzo, cyclohexyl and bicyclic rings, the hexahydropyridine-1,3-dione ring system, and the cyclized R⁸⁻R⁹ rings may all be optionally substituted.

Compounds contemplated as falling within the scope of the present invention include, but are not limited to the following examples:
2R-(1,4-Benzodioxanyl-2-methyl)-4-methoxy-1,2,3,2a,8,8a-hexahydroindeno[1,2-c] pyrrole;
2S-(1,4-Benzodioxanyl-2-methyl)-4-methoxy-1,2,2,3a, 8,8a-hexahydroindeno[1,2-c]pyrrole;
2-(3-(3,3-Tetramethylene)glutarimidyl)propyl)-4-hydroxy-1,2,3,3a,8,8a-hexahydroindeno-[1,2-c]pyrrole;
2-(3-(3,3-Tetramethylene)glutarimidyl)propyl)-4-methoxy-1,2,3,3a,8,8a-hexahydroindeno-[1,2-c]pyrrole;
2-(4-(3,3-Tetramethylene)glutarimidyl)butyl-4-hydroxy-1,2,3,3a,8,8a-hexahydroindeno-[1.2-c]pyrrole;
2-(4-(3,3-Dimethyl)glutarimidyl)butyl-4-methoxy-1,2,3,3a_{,}8,8a-hexahydroindeno[1.2-c]pyrrole;
2-(4-(3,3-Tetramethylene)glutarimidyl)butyl-4-methoxy-1,2,3, 3a,8,8a-hexahydroindeno-[1,2-c]pyrrole;
2-(4-(3,3-Tetramethylene)glutarimidyl)butyl-5-chloro-4-hydroxy-1,2,2,3a,8,8a-hexahydro-indeno[1.2-c]pyrrole;
2-(4-(3,3-Tetramethylene)glutarimidyl)butyl-5-chloro-4-methoxy-1,2,2,3a,8,8a-hexahydro-indeno[1.2-c]pyrrole;
2-(5-(3,3-Tetramethylene)glutarimidyl)pentyl-4-methoxy-1,2,3,3a,8,8a-hexahydroindeno[1,2-c]pyrrole;
2-(4-(3,3-Tetramethylene)glutarimidyl)cis-buten-2-yl-4-methoxy-1,2,2,3a,8,8a-hexahydroindeno[1,2-c]pyrrole;
2-(4-(3,3-Tetramethylene)glutarimidyl)trans-buten-2-yl-4-methoxy-1,2,3,3a,8,8a-hexahydroindeno[1,2-c]pyrrole;
4-Hydroxy-2-(2-(4-Fluorobenzamido)ethyl)-1,2,3,3a,8,8a-hexahydroindeno[1,2-c]pyrrole;
4-Methoxy-2-(2-(4-Fluorobenzamido)ethyl)-1,2,2,3a,8,8a-hexahydroindeno[1,2-c]pyrrole;
4-Hydroxy-2-(4-(2-(1,2-Benzoisothiazolin-3-one-1,1-dioxide))butyl)-1,2,3,3a,8,8a-hexahydroindeno[1,2-c]-pyrrole;
4-Methoxy-2-(4-(2-(1,2-Benzoisothiazolin-3-one-1,1-dioxide))butyl)-1,2,3,3a,8,8a-hexahydroindeno[1,2-c]-pyrrole;
4-Hydroxy-2-(4-(2-phthalimido)butyl)-1,2,3,3a,8,8a-hexahydroindeno-[1,2-c]pyrrole;
4-Methoxy-2-(4-(2-phthalimido)butyl)-1,2,3,3a,8,8a-hexahydroindeno-[1,2-c]pyrrole;
cis-2-(3,3-Tetramethylene)glutarimidyl)propyl-5-methoxy-2,3,4,4a,9,9a-hexahydro-1H-indeno[2,1-c]pyridine;
trans-2-(3,3-Tetramethylene)glutarimidyl)propyl-5-methoxy-2,3,4,4a,9,9a-hexahydro-1H-indeno[2,1-c]pyridine;
cis-2-(3,3-Tetramethylene)glutarimidyl)butyl-5-methoxy-2,3,4,4a,9,9a-hexahydro-1H-indeno[2,1-c]pyridine;
trans-2-(3,3-Tetramethylene)glutarimidyl)butyl-5-methoxy-2,2,4,4a,9,9a-hexahydro-1H-indeno[2,1-c]pyridine;
trans-2(3-(2-(1,2-benzisothiazolin-3-one-1,1-dioxide))propyl)-5-methoxy-2,3,4,4a,9,9a-hexahydro-1H-indeno [2,1-c]pyridine;
trans-2(4-(2-1,2-benzisothiazolin-3-one-1,1-dioxide))butyl)-5-methoxy-2,3,4,4a,9,9a-hexahydro-1H-indeno [2,1-c]pyridine;
trans-2-(2-(4-fluorobenzamido)ethyl)-5-methoxy-2,3,4,4a,9,9a-hexahydro-1H-indeno[2,-c]pyridinehydrochlo- ride;

As used throughout this specification and the appended claims, the term "optionally substituted" shall mean a group or radical that is substituted with halogen, lower alkyl, mono- or di(lower alkyl)-substituted lower alkyl, (lower alkyl)thio, halo-substituted lower alkyl amino-substituted lower alkyl, mono- or di(lower alkyl)-substituted amino, lower alkenyl, lower alkynyl, lower alkoxy, aryloxy, aryl(lower alkyl), hydroxy, cyano, amino, mono- and di(lower alkyl)amino, or nitro.

The term "lower alkyl" refers to branched or unbranched saturated hydrocarbon radicals having from one to six carbon atoms. Representatives of such groups are methyl, ethyl, propyl, iso-propyl, butyl, sec-butyl, iso-butyl, tert-butyl, pentyl, neo-pentyl, iso-pentyl, tert-pentyl and isohexyl.

The term "alkenyl" denotes a branched or. unbranched monovalent hydrocarbon radical containing at least one carbon-carbon double bond, such as ethenyl (-CH=CH₂) and propenyl (-CH₂-CH=CH₂).

The term "alkenylene" refers to a divalent branched or unbranched hydrocarbon radical containing at least one carbon-carbon double bond. Examples include ethenylene(-CH=CH-) and propenylene (-CH₂CH=CH-).

The term "alkynyl" refers to a monovalent branched or unbranched hydrocarbon radical containing at least one carbon-carbon triple bond, for example ethynyl and propynyl.

The term "lower alkoxy" denotes an alkyl group as defined above, attached to the parent molecular moiety through an oxygen atom. Representatives of such groups include methoxy, ethoxy and butyoxy.

The term "(lower alkyl)thio" refers to a lower alkyl group as defined above, attached to the parent molecular moiety through a sulfur atom. Typical (lower alkyl)thio groups include methylthio, ethylthio, propylthio and iso-propylthio.

The terms "halo-" and "halogen" refer to a substituent selected from fluoro-, chloro-, bromo-, and iodo-.

The term "aryl" as used herein refers to mono- or polycyclichydrocarbon fused or nonfused aromatic ring systems which may contain one or more hetero atoms such as oxygen, nitrogen, or sulfur in the ring system and which may be optionally substituted as defined hereinabove. Representative aryl groups are phenyl, naphthyl, biphenylene, triphenyl, pyridyl, pyrrolyl, furyl, thienyl, indolyl, pyrazyl, iso-quinolyl, thiazolyl, imidazolyl, oxazolyl, puryl, phenazyl and carbazolyl.

The term "arylalkyl" as used herein refers to a substituted or unsubstituted aryl ring system as defined above attached to a lower alkyl radical as defined above Such groups include, but are not limited to benzyl and other arylalkyl groups in which the aryl portion is quinolyl, thiazolyl, imidazolyl, oxazolyl, puryl, phenazyl and carbazolyl.

The term "alkylenedioxy" as used herein refers to a divalent radical of the form -O-X-O- wherein X is methylene (-CH₂-), ethylene (-CH₂CH₂-), trimethylene (-CH₂CH₂CH₂-), carbonyl (>C=O), or sulfonyl (>S=O).

The term "benzoalkylenedioxoalkylene" as used herein refers to the structure 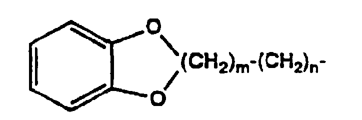 where m and n are independently 1 or 2.

The terms "arylamidoalkenylene" and "arylamidoalkylene" refer, respectively, to the structures aryl-CO-NH-(alkenylene)- and aryl-CO-NH-(alkylene)- wherein aryl, alkenylene, and alkylene are as defined above.

The terms "aryl(lower alkyl)amidoalkenylene" and "aryl(lower alkyl)amidoalkylene" refer, respectively, to groups having the structures aryl(lower alkyl)-CO-NH-alkenylene and aryl(lower alkyl)-CO-NH-alkylene, with aryl, alkylene, and alkenylene as defined above

The terms "bicyclo" and "bicyclic ring" refer to groups of the formula 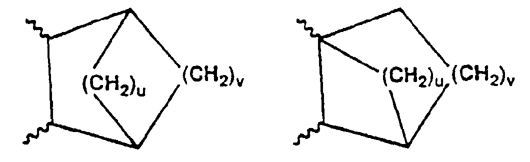 where u and v are independent integers having a value of from 1 to 3.

The terms "bivalent aliphatic hydrocarbon" and "divalent aliphatic hydrocarbon" refer to radicals derived from saturated and unsaturated, branched and unbranched acyclic hydrocarbons of from one to twelve carbon atoms and having two free valence bonds. Such groups include methylene, ethylene, propylene, methylethylene, ethylethylene and 4-methyl-2-pentylene.

The term "electron withdrawing groups" as used herein means substituent groups which have an inductive electron withdrawing effect greater than that of hydrogen. Hammet substituent sigma constants for various substituent groups, calculated for the ionization of meta-substituted benzoic acids provide a useful means of predicting relative electron withdrawing inductive effects, and are well known to those familiar with physical organic chemistry. See, for example, Hine, Physical Organic Chemistry, McGraw-Hill Book Co., New York, 1962_{;} pp. 85-88.

Those substituent groups having a greater electron-withdrawing inductive effect than hydrogen have positive Ham- melt sigma constants. Those substituents which exhibit a lesser inductive electron-withdrawing effect than hydrogen possess negative sigma constants.

Representative electron-withdrawing substituents are halo, mercapto, acylmercapto such as acetylmercapto, alkyl- sulfido such as methylsulfido (CH₃S-), nitro, cyano, keto groups such as acetyl, halomethyl such as chloromethyl, and alkoxyalkyl groups such as methoxymethyl (CH30CH2-)

The term "pharmaceutically aceptable salts" refers to the relatively non-toxic, inorganic and organic acid addition salts and, where the compounds of this invention also contain an acidic functional group, the alkali and alkaline earth metal salts. These salts can be prepared in situ during the final isolation and purification of the compounds or by separately reacting the purified compound in its free base form with a suitable organic or inorganic acid and isolating the salt thus formed. Representative salts include the hydrobromide, hydrochloride, sulfate, bisulfate, phosphate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate and laurylsulpho- nate salts.

Representative alkali or alkaline earth salts include the sodium, potassium, calcium, and magnesium salts.

Certain of the compounds of this invention may exist in cis-/trans-isomeric forms and/or possess one or more chiral centers and may exist in optically active forms. This invention contemplates all isomeric forms of the compounds including cis-/trans-isomers as well as individual optical isomers and mixtures thereof.

The present invention also provides pharmaceutical compositions which comprise one or more of the compounds of formula I above formulated together with one or more non-toxic pharmaceutically acceptable carriers. The pharmaceutical compositions may be specially formulated for oral administration in solid or liquid form, for parenteral injection, or for rectal administration.

The pharmaceutical compositions of this invention can be administered to humans and other animals orally rectally, parenterally (i.e. intravenously, intramuscularly, or sub-cutaneously), intracisternally, intravaginally, intraperitoneally, topically (as by powders, ointments, or drops), bucally, or as an oral or nasal spray.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous cariers, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like, Prolonged absorption of the injectable pharmaceutical form may be brought about by the inclusion of agents which delay abdorption such as aluminum monostearate and gelatin.

If desired, and for more effective distribution, the compounds can be incorporated into slow release or targeted delivery systems such as polymer matrices, liposomes, and microspheres.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alignates, gelatin, polyvinylpyrrolidone, surcrose, and acacia, c) humectants such as glycerol, d) disintegrating agents such as agar-agar, calcium carbonate, potato ortapioca starch, alginic acid, certain silicates, and sodium carbonate, e) solution retarding agents such as paraffin, f) absorption accelerators such as quaternaryammonium compounds, g) wetting agents such as, for example, cetyl alcohol and glycerol monostearate, h) absorbents such as kaolin and bentonite clay and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above- mentione excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose aluminum metahydroxide, bentonite, agar-agar, and tragacanth, and mixtures thereof.

Compositions for rectal or vaginal administration are preferably suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Dosage forms for topical administration of a compound of this invention include powders, sprays, ointments and inhalants. The active compound is mixed under sterile conditions with a pharmaceutically acceptable carrier and any needed preservatives, buffers, or propellants which may be required. Opthalmic formulations, eye ointments, powders and solutions are also contemplated as being within the scope of this invention.

Actual dosage levels of active ingredients in the pharmaceutical compositions of this invention may be varied so as to obtain an amount of the active compound(s) that is effective to achieve the desired therapeutic response for a particular patient, compositions, and mode of administration. The selected dosage level will depend upon the activity of the particular compound, the route of administration, the severity of the condition being treated, and the condition and prior medical history of the patient being treated. However, it is within the skill of the art to start doses of the compound at levels lower than required for to achieve the desired therapeutic effect and to gradually increase the dosage until the desired effect is achieved.

Generally dosage levels of about 0.1 to about 200, more preferably of about 0.5 to about 150, and most preferably about 1 to about 125 mg of active compound per kilogram of body weight per day are administered orally to a mammalian patient suffering from hypertension. If desired, the effective daily dose may be divided into multiple doses for purposes of administration, e.g. two to four separate doses per day.

The compounds of this invention are prepared by the methods generally depicted in Reaction Schemes I and 11 below.

Referring to Reaction Scheme I, the ester 1 is treated with potassium t-butoxide in ether, followed by condensation with diethyl oxalate to yield the keto-diester 2. Cyclization of 2 in PPA at room temperature affords the unsaturated diester 3, which upon catalytic hydrogenation yields the diester 4. The diester is converted to the N-benzyl diamide by treatment with excess of a 1:1 complex of trimethyl aluminum and benzyl amine, which upon treatment with one equivalent of para-toluenesulfonic acid in refluxing xylene yields the imide 5. Borane reduction to the tertiary amine (6) followed by hydrogenolysis yields the key intermediate 7. This intermediate is treated with variously substituted alkyl bromides in acetonitrile in the presence of ethyl diisopropyl amine to yield the final produces (8) described in this patent application.

### Scheme

### Synthesis of 4-Alkoxy-1,2,3,3a,8,8a-hexahydroindeno[1,2-c]pyrroles

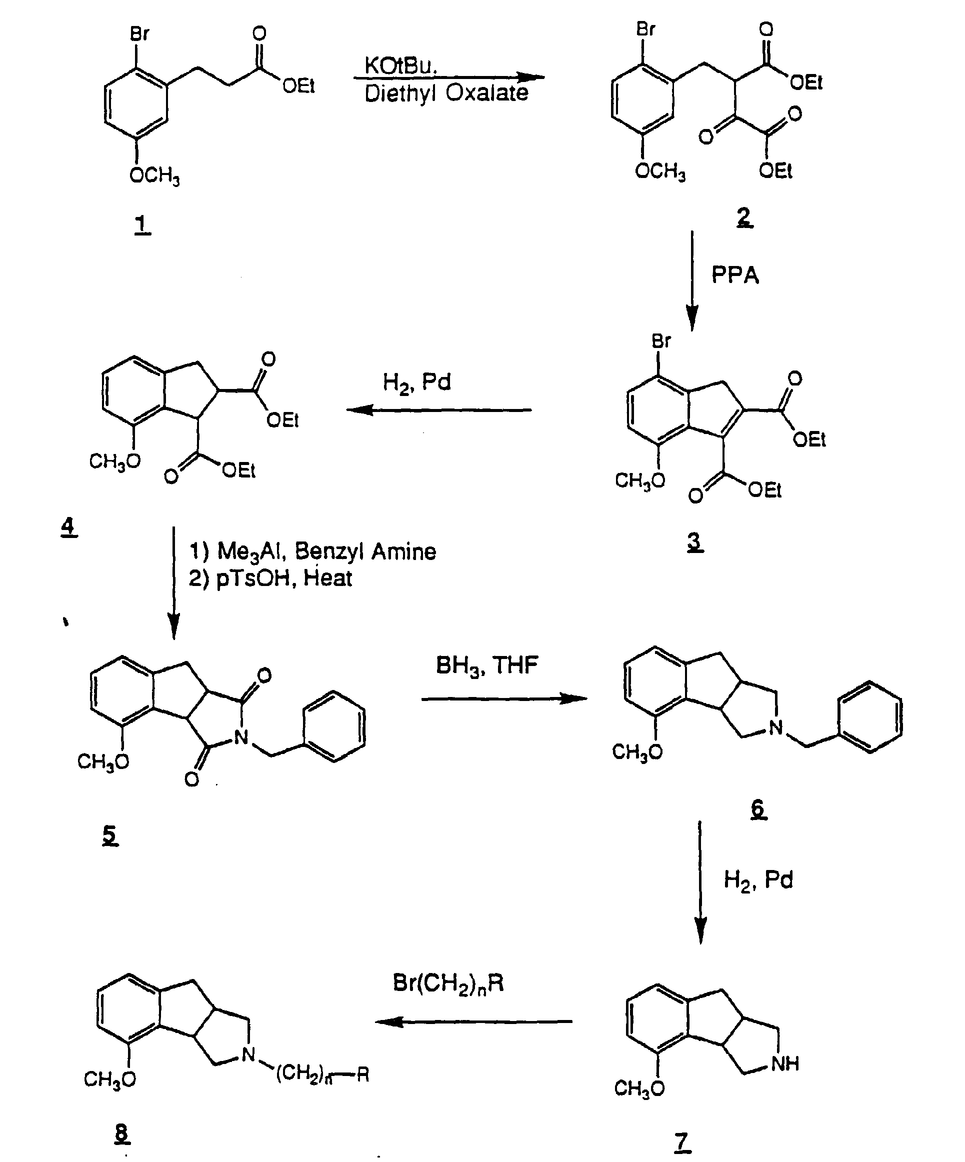

Referring to Reaction Scheme II, cyclization of the acid 1 in polyphosphoric acid at 90° C. yields the indanone 2. Treatment of the indanone with triethyl orthoformate in the presence of boron trifluoride etherate, followed by ethyl diisopropyl amine yields the acetal 3. Modified Peterson olefination of 3 yields the unsaturated ester 4, which upon treatment with Pd/C and H₂ yields intermediate 5. Hydrolysis of the acetal, followed by AgO oxidation and esterification yields the diester 7. The diester is converted to the N-benzyl diamide by treatment with an excess of a 1:1 complex of benzyl amine and trimethyl aluminum, which upon treatment with 1 equivalent of para-toluenesulfonic acid in refluxing xylene yields the imide 8 as a mixture of cis- and trans-isomers, which are separated. Reduction of the purified imide isomers yields the cis- and trans-isomers of 9, which upon treatment with H₂/Pd yields the cis- and trans-isomers of the key intermediate 10. Alkylation with variously substituted alkyl halides yields the final products (11) described in this patent application.

### Scheme II

### Synthesis of 5-Alkoxy-2,3,4,4a,9,9a-hexahydro-1 H-Indeno[2,1-c]pyridines

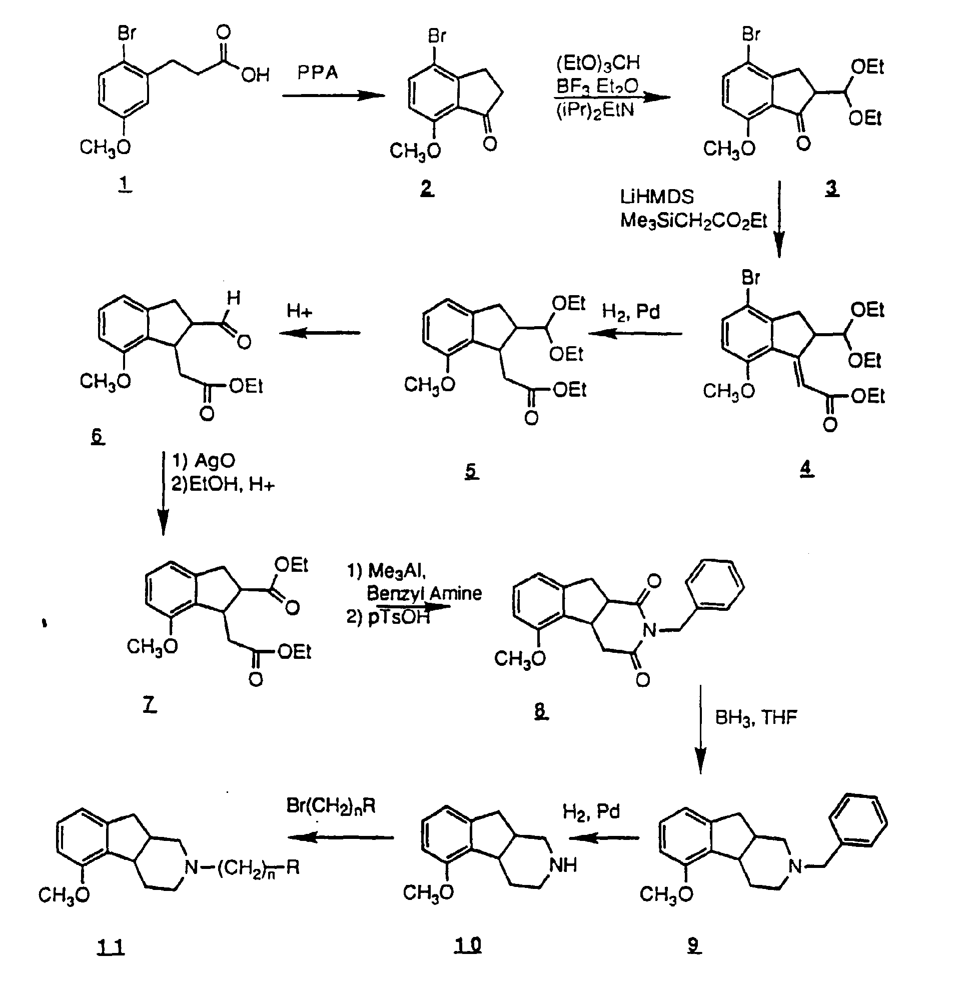

The following examples are provided to enable one skilled in the art to practice the present invention. These examples, however, are merely illustrative of the invention, and are not to be read as limiting the scope of the invention as it is defined by the appended claims/

Examples 5,6, 9-15, 33, 41, 42 and 45-49 are not compounds of the invention but are included to illustrate the synthetic methods.

### Example 1

### Preparation of 2-Oxo-3-carboethoxy-4-(2-bromo-5-methoxyphenyl)butanoic acid, ethyl ester

Potassium t-butoxide (6.72 g) was suspended in 30 ml of anhydrous ether and cooled to 0° C. A solution of 14.35 g of ethyl 3-(2-bromo-5-methoxyphenyl)propionate and 10.95 g diethyl oxalate in 10 ml ether was added dropwise over 15 min. After 2h at 25° C., the reaction was poured into 100 ml H₂0 and the aqueous layer was separated, acidified to pH 1 and extracted with ether. The ether layer was dried (MgS0₄) and the solvent was evaporated to yield 17.48 g of the desired product as a colorless oil (90%). NMR (CDCI₃) 8 1.20 (t, 3H), 1.35 (t, 3H), 3.32 (dd, 2H), 3.76 (s, 3H), 4.18 (q, '2H), 4.32 (q, 2H), 4.53 (t, IH), 6.67 (dd, 1 H), 6.80 (d, 1 H), 7.40 (d, 1 H).

### Example 2

### Preparation of 7-Bromo-4-methoxy-1H-indene-2,3-dicarboxilic acid, diethyl ester

The ester from Example 1 (25.0 g) was added to 250 g polyphosphoric acid, and the reaction was stirred at 25° C for 45 min. The reaction was poured into 1.5 kg ice/H₂0 and extracted with ether. The ether extracts were washed with 5% NaHC0₃ solution, brine, dried (MgS0₄) and evaporated to dryness. The resulting product was triturated with hot hexane, cooled, and filtered to yield 13.98 g of the desired product as a white solid, mp: 99-101° C. NMR (CDCl₃) δ 1.34 (t, 3H), 1.41 (t, 3H), 3.71 (s, 2H), 3.83 (s, 3H), 4.29 (q, 2H), 4.44 (q, 2H), 6.73 (d, 1 H), 7.42 (d, 1 H).

### Example 3

### Preparation of 4-Methoxy-2,3-dihydro-1H-indene-2,3-dicarboxylic acid, diethyl ester

The diester from Example 2 (19.39 g) was dissolved in 250 ml ethanol. To the solution was added 5.20 g of 10% Pd/BaS0₄ and 7.15 g NaOAC·3H₂O. The reaction was hydrogenated at 4 atm pressure. The catalyst was filtered and the solvent evaporated. The product was dissolved in ether, washed with 5% NaHC0₃, dried (MgS0₄), and the solvent was evaporated to yield 14.87 g of the desired product as a colorless oil. NMR (CDCl₃) δ 1.20 (t, 3H), 1 27 (t, 3H), 3.13 (dd, 1H), 3.48-3.56 (m, 2H), 3.82 (s, 3H), 3.95-4.22 (m, 4H), 4.39 (d, 1H), 6.70 (d, 1 H), 6.87 (d, 1 H), 7.21 (t, 1 H).

### Example 4

### Preparation of 2-Benzyl-4-methoxy-1,2,3,3a,8,8a-hexahydroindeno[1,2-c]pyrrole-1,3-dione

Benzyl amine (8.02 g) was dissolved in 125 ml toluene. To the solution was added 37.5 ml of a 2.0M solution of Me₃AI in toluene. After 20 min., 7.30 g of the diester from example 3 was added. The reaction was heated at reflux for 2 hours, cooled to 0° C and quenched by the dropwise addition of 15 ml H₂0. The reaction was poured in to 5% HCl solution and extracted with ethyl acetate. The organic extracts were washed with brine, dried (MgSO₄) and evaporated to dryness. The resulting product was dissolved in 400 ml xylene and 5.22 g pTSOH.H₂0 and heated at reflux for 18 h. The solvent was evaporated and the product was recrystallized from methanol to yield 6.10 g of the desired product as a white solid, mp: 170-2° C. NMR (CDCl₃) 8 3.37 (dd, 2H), 3.63 (m, 1 H), 3.91 (s, 3H), 4.52 (d, 1 H), 4.60 (dd, 2H), 6.77 (d, 1H), 6.73 (d, 1H), 7.21-7.37 (m, 6H).

### Example 5

### Preparation of 2-Benzyl-4-methoxy-1,2,3,3a,8,8a-hexahydroindeno[1,2-c]pyrrole hydrochloride

The product from Example 4 (5.76 g) was dissolved in 200 ml tetrahydrofuran (THF) and to the solution was added 2.88 g LiAIH₄. The reaction was stirred at reflux for 3h, and then cooled to 25° and quenched by the addition of 2.9 ml H₂0, 2.9 ml 15% NaOH, 8.7 ml H₂0. The reaction was filtered and the solvent evaporated to dryness. The product was dissolved in diethyl ether and treated with an ethereal solution of HCI, evaporated to dryness, and then recrystallized from CH₂Cl₂/EtOAc to yield 4.90 g of the desired product as a white solid, mp: 222-3° C. NMR (d₆ DMSO) δ 2.7-3.9 (m, 8H), 3.77 (s, 3H), 4.33 (dd, 2H), 6.78, (d, 2H), 6.83 (d, 2H), 7.23 (t, 1H), 7.40-7.60 (m, 5H)

### Example 6

### Preparaion of 4-Methoxy-1,2,3,3a,8,8a-hexahydroindeno[1,2-c]pyrrole hydrochloride

The product from Example 5 (4.90 g) was hydrogenated with 2.45 g 20% Pd/C in 200 ml methanol at 3 atm, affording 3.17 g of the desired product, mp: 208-209° C. NMR (d₆ DMSO/D₂O) 8 2.83 (m, 2H), 3.14-3.29 (m, 3H), 3.46 (dd, 1 H), 3.55 (dd, IH), 3.80 (s, 3H), 3.95 (m, 1 H), 6,85 (d, 2H), 7.25 (t, 1 H).

Analysis, theoretical (1/4 H₂0): C, 62.61; H, 7.22; N, 6.08. Found: C, 62.24; H, 7.00; N, 5.87.

### Example 7

### Preparation of 2-(4-(3,3-tetramethylene)glutarimidyl)butyl-4-methoxy-1,2,3,3a,8,8a-hexahydoindeno[1,2-c]pyrrole hydrochloride

The product from Example 6 (0.902 g) was combined with 1.33 g 1-bromo-4-(3,3-tetramethyleneglutarimidyl)-butane and 2.1 ml diisopropyl ethyl amine in 10 ml acetonitrile. After 18 h at reflux, the reaction was quenched in aqueous 5% NaHC0₃ solution and extracted with diethyl ether. The ether extracts were dried (K₂CO₃) and evaporated. The resulting oil was treated with ethereal HC1, and the resulting hydrochloride salt was recrystallized from EtOAc/ether to yield 1.15 g of a white solid, mp: 117-22° C. NMR (d₆ DMSO) δ 1.40 (m, 6H), 1.53 (m, 6H), 2.62 (s, 4H), 2.70-3.50 (m, 10H), 3.62 (t, 2H), 3.80 (s, 3H), 6.83 (d, 2H), 7.23 (t, 1 H).

Analysis, theoretical (with 1 H₂O) : C, 64.57; H, 8.02; N, 6.02. Found: C, 64.96; H, 7.66; N, 6.01.

### Example 8

### Preparation of 2-(4-(3,3-tetramethylene)glutarimidyl)butyl-5-chroro-4-methoxy-1,2,3,3a,8,8a-hexahydroindeno[1,2-c] pyrrole hydrochloride

The product from Example 7 (0.446 g) was dissolved in 5 ml methylene chloride and 1 ml acetic acid. The solution was cooled to 0° C and a solution of Cl₂ in acetic acid (1 ml of a 1.0 M solution) was added. After 15 min, the solvent was evaporated, and the resulting product was recrystallized from ethyl acetate and ether to yield 0.410 g of a white solid, mp: 157-60° C. NMR (d₆ DMSO) δ 1.40 (m, 6H), 1.60 (m, 6H), 2.60 (s, 4H), 2.60-3.40 (m, 12H), 3.60 (t, 2H), 3.80 (s, 3H), 3.97 (m, 2H), 6.90 (d, 1 H), 7.30 (d, 1 H).

Analysis, theoretical: C, 62.37; H, 7.12; N, 5.82. Found: C, 62.77; H, 7.23; N, 5.73.

### Example 9

### Preparation of 4-Methoxy-2-propyl-1,2,3,3a,8,8a-hexahydroindeno[1,2-c]pyrrole hydrochloride

The product from Example 6 (1.00 g) was dissolved in 100 ml ethanol. To the reaction was added 2 ml propionaldehyde and 100 mg 10% Pt/C. The reaction was hydrogenated for 4 h at 3 atm, filtered, and evaporated. The resulting product was recrystallized from ethanol and ether to yield 0.825 g of the desired product, mp: 161-3° C. NMR (dₛ DMSO) δ 0.88 (t, 3H), 1.62 (m, 2H), 2.55-3.60 (m, 8H), 3.80 (s, 3H), 3.80-4.08 (m, 2H), 6.82 (d, 2H), 7.23 (t, 1 H).

Analysis, theoretical: C, 67.28; H, 8.28; N, 5.15. Found: C, 66.73; H, 8.26; N, 5.15.

### Example 10

### Prepatation of 4-Hydroxy-2-propyl-1,2,3,3a,8,8a-hexahydroindeno[1,2-c]pyrrole hydrobromide

The product from Example 9 (0.490 g) was dissolved in 25 ml methylene chloride and cooled to -78° C. To the reaction was added 0.69 ml BBr₃. The reaction was warmed to 0° C. for 4 h and then cooled to -78° C. The reaction was quenched by the addition of 5 ml methanol and the solvent was evaporated. The resulting solid was recrystallized from ethanol and ether to yield 0.470 g white solid, mp: 187-9° C NMR (dₛ DMSO) 8 0.88 (t, 3H), 1.65 (m, 2H), 2.60-4.10 (m, 10H), 6.65 (d, 1 H), 6.70 (d, 1 H), 7.05 (t, 1 H).

Analysis, theoretical: C, 56.39; H, 6.76; N, 4.70. Found: C, 56.42; N, 6.53; N, 4.65.

### Example 11

### Preparation of 4-Hydroxy-1,2,3,3a,8,8a-hexahydroindeno[1,2-c]pyrrole hydrobromide

The product from Example 6 (0.226 g) was treated as described in Example 10 to yield 0.204 g of a white solid, mp: 221-2° C. NMR (d₆ DMSO) δ 2.83 (m, 2H), 3.10-3.60 (m, 5H), 3.92 (m, 1 H), 6.67 (d, 1 H), 6.70 (d, 1 H), 7.07 (t, 1 H).

Analysis, theoretical (1/4 H₂0) : C, 50.69; H, 5.61; N, 5.37. Found: C, 50.61; H, 5.45; N, 5.22.

### Example 12

### Preparation of 4-Methoxy-2-methyl-1,2,3,3a,8,8a-hexahydroindeno[1,2-c]pyrrole hydrochloride

The product from Example 6 (0.600 g) was dissolved in 15 ml methanol and 8 ml 37% aqueous formaldehyde. The solution was stirred with 0.80 g NaBH₃CN for 18 h. The reaction was quenched in H₂0, basified to pH 10, and extracted with ether. The ether extracts were dried, treated with ethereal HCI, and evaporated. The resulting white solid was recrystallized from ethanol and ether to yield 0.355 g of a white solid, mp: 184-5° C. NMR (dδ DMSO) δ 2.75 (s, 3H), 2.55-4.05 (m, 8H), 3.80 (s, 3H), 6.82 (d, 2H), 7.22 (t, 1 H).

Analysis, theoretical (1/8 H₂0): C, 64.53; H, 7.60; N, 5.78. Found: C, 64.48; H, 7.44; N, 5.70.

### Example 13

### Preparation of 4-Methoxy-2-ethyl-1,2,3,3a,8,8a-hexahydroindeno[1,2-c]pyrrole hydrochloride

The product from Example 6 (0.580 g) was dissolved in 50 ml ethanol. To the reaction was added 2 ml acetaldehyde and 100 mg 10% Pt/C. The reaction was hydrogenated for 4 h at 3 atm, filtered, and evaporated. The resulting product was recrystallized from ethanol and ether to yield 0.501 g of the desired product, mp: 161-3° C. NMR (d₆ DMSO) δ 1.30 (m, 3H), 2.55-3.60 (m, 8H), 3.80 (s, 3H), 3.80-4.08 (m, 2H), 6.82 (m, 2H), 7.22 (t, 1 H).

Analysis, theoretical: C, 66.26; H, 7.94; N, 5.52. Found: C, 65.74; H, 7.86; N, 5.31.

### Example 14

### Preparation of 4-Hydroxy-2-methyl-1,2,3,3a,8,8a-hexahydroindeno[1.2-c]pyrrole hydrobromide

The product from Example 12 (0.479 g) was treated as described in Example 10 to yield 0.465 g of the desired product, mp: 213-15° C. NMR (d₆ DMSO) δ 2.60-3.50 (m, 5H), 3.35 (s, 3H), 3.60-4.10 (m, 3H), 6.65 (m, 2H), 7.07 (t, 1H). Analysis, theoretical (1/4 H₂0): C, 52.47; H, 6.05; N, 5.10. Found: C, 52.61; H, 5.78; N, 5.03.

### Example 15

### Preparation of 4-Hydroxy-2-ethyl-1,2,3,3a,8,8a-hexahydroindeno[1,2-c]pyrrole hydrobromide

The product from Example 13 (0.350 g) was treated as described in Example 10 to yield 0.358 g of the desired product, mp: 168-70° C. NMR (d₆ DMSO) δ 1.20 (t, 3H), 2.50-4.10 (m, 10H), 6.66 (d, 1 H), 6.69 (d, 1 H), 7.07 (t, 1 H). Analysis, theoretical (1/4 H₂0): C, 54.09; H, 6.46; N, 4.85. Found: C, 54.16; H, 6.27; N, 4.66.

### Example 16

### Preparation of 4-Methoxy-2-cyanomethyl-1,2,3,3a,8,8a-hexahydroindeno[1,2-c]pyrrole

The product from Example 6 (0.895 g) was dissolved in 4 ml acetonitrile and 4 ml diisopropyl ethyl amine. 0.270 ml chloroacetonitrile was added and the reaction was stirred at 60° C. for 18 h. The reaction was quenched in 5% aqueous NaOH and extracted with methylene chloride, dried (K₂CO₃), and evaporated to yield 0.937 g of the desired product as a white solid. NMR (CDCl₃) δ 2.56 (dd, 1 H), 2.81 (m, 1 H), 2.89 (m, 2H), 3.02 (t, 1 H), 3.12 (m, 1 H), 3.29 (t, 1 H), 3.62 (s, 2H), 3.81 (s, 3H), 3.89 (m, 1H), 6.66 (d, 1H), 6.78 (d, 1H), 7.17 (t, 1 H).

### Example 17

### Preparation of 4-Methoxy-2-(2-aminoethyl)-1,2,3,3a,8,8a-hexahydroindeno[1,2-c]pyrrole

The product from Example 16 (1.00 g) was dissolved in 90 ml methanol and 10 ml anhydrous ammonia. 0.100 g 5%Rh/Al₂O₃ was added and the reaction was hydrogenated at 4 atm for 4h. The reaction was filtered and the solvent removed to yield 0.97 g of the desired product as a colorless oil. NMR (CDCl₃) δ 2.20-2.61 (m, 3H), 2.66-3.45 (m, 8H), 3.82 (s, 3H), 3.89 (m, 1 H), 6.67 (d, 1 H), 6.78 (d, 1 H), 7.14 (t, 1 H).

### Example 18

### Preparation of 4-Methoxy-2-(2-(4-fluorobenzamido)ethyl)-1,2,3,3a,8,8a-hexahydroindeno[1,2-c]pyrrole hydrochloride

The product from Example 17 (0.95 g) was dissolved in 50 ml methylene chloride and 1.5 ml triethyl amine. The solution was cooled to -20° C. and 0.59 ml p-fluorobenzoyl chloride was added. After 30 min, the reaction was quenched in 5% NaHC0₃ and extracted with ethyl acetate. The organic extracts were dried (Na₂S0₄), evaporated, and the product was dissolved in ether and treated with ethereal HCl and then evaporated to dryness. The resulting product was recrystallized from ethyl acetate and ether to yield 0.772 g of the desired product as a white solid, mp: 197-8° C. NMR (d₆ DMSO) δ 2.60-3.10 (m, 3H), 3.00-3.50 (m, 4H), 3.59 (m, 2H), 3.78 (s, 3H), 3.82-4.22 (m, 3H), 6.83 (d, 1 H), 6.87 (d, 1 H), 7.23 (t, 1 H), 7.34 (m, 2H), 8.00 (m, 2H).

Analysis, theoretical: C, 64.53; H, 6.19; N, 7.17. Found: C, 64.40; H, 6.20; N, 7.07.

### Example 19

### Preparation of 4-Hydroxy-2-(2-(4-fluorobenzamido)ethyl)-1,2,3,3a,8,8a-hexahydroindeno[1,2-c]pyrrole hydrobromide

The product from Example 18 (0.323 g) was treated as described in Example 10 to yield 0.347 g of the desired product as a white solid, mp: 144-7° C. NMR (d₆ DMSO) 8 2.70-4.40 (m, 12H), 6.68 (m, 2H), 7.07 (t, 1 H), 7.35 (m, 2H), 7.97 (m, 2H).

Analysis, theoretical (1/2 H₂0): C, 55.82; H, 5.39; N, 6.51. Found: C, 55.68; H, 5.20; N, 6.45.

### Example 20

### Preparation of 2-(4-(3,3-tetramethylene)glutarimidyl)butyl-4-hydroxy-1,2,3,3a,8,8a-hexahydroindeno[1,2-c]pyrrole hydrobromide

The product from Example 7 (0.446 g)was treated as described in Example 10 to yield 0.359 g of the desired product as a white solid, mp: 167-9° C. NMR (d₆ DMSO) 8 1.40 (m, 6H), 1.62 (m, 6H), 2.63 (s, 4H), 2.70-3.45 (m, 8H), 3.61 (t, 2H), 3.70-4.10 (m, 2H), 6.65 (m, 2H), 7.03 (t, 1H). Analysis, theoretical (1/2 H₂0): C, 65.22; H, 7.75; N, 6.34. Found: C, 65.41; H, 7.45; N, 6.34.

### Example 21

### Preparation of 4-Methoxy-2-(4-(2-(1,2-benzisothiazolin-3-one1,1-dioxide))butyl)1,2,3,3a,8,8a-hexahydroindeno -

### [1,2-c]pyrrole hydrochloride

The product from Example 6 (0.902 g) was dissolved in 10 ml acetonitrile and 2 ml diisopropyl ethyl amine. To the reaction was added 1.59 g 4-bromobutyl-2-(1,2-benzisothiazolin-3-one-1,1-dioxide, and the reaction was heated at reflux for 4 h. The reaction was quenched in 5% NaHC0₃, extracted with ether dried (K₂CO₃), and evaporated to dryness. The product, after chromatography, was treated with ethereal HCl, and then recrystallized from ethanol and ether to yield 1.08 g of the desired product as a white solid, mp: 175-8° C. NMR (d₆ DMSO) δ 2.50-4.10 (m, 16H), 3.80 (s, 3H), 6.82 (m, 2H), 7.22 (t, 1 H), 8.07 (m, 3H), 8.31 (m, 1 H).

Analysis, theoretical: C, 59.67; H, 5.88; N, 6.05. Found: C, 59.47; H, 5.98; N, 6.01.

### Example 22

### Preparation of 4-Hydroxy-2-(4-(2-(1.2-benzisothiazolin-3-one-1,1-dioxide))butyl)-1,2,3,3a,8,8a-hexahydroindeno-[1,2-c]pyrrole hydrochloride

The product from Example 21 (0.462 g) was treated as described in Example 10 to yield 0.309 g of the desired product as a white solid, mp: 218-20° C. NMR (d₆ DMSO) δ 2.55-3.50 (m, 10H), 3.60-4,15 (m, 6H), 6.68 (m, 2H), 7.05 (t, 1 H), 8.10 (m, 3H), 8.32 (d, 1H).

Analysis, theoretical: C, 53.55; H, 5.11; N, 5.68. Found: C, 53.11; H, 5.01; N, 5.61.

### Example 23

### Preparation of 2-(4-(3,3-dimethyl)glutarimidyl)butyl-4-methoxy-1,2,3,3a,8,8a-hexahydroindeno-[1,2-c]pyrrole hydrochloride

The product from Example 6 (0.60 g) was combined with 0.82 g. 1-bromo-4-(3,3-dimethylglutarimidyl)-butane and 1.4 ml diisopropyl ethyl amine in 10 ml acetonitrile. After 18 h at reflux, the reaction was quenched in aqueous 5% NaHC0₃ solution and extracted with diethyl ether. The organic layer was washed with brine, dried (K₂CO₃) and evaporated. The resulting oil was treated with ethereal HCI, and the resulting hydrochloride salt was recrystallized from EtOAc/ether to yield 0.58 g of a white solid, mp: 143-146° C. NMR ( d₆ DMSO) 8 0.95 (s, 5H), 1.40 (q, 2H), 1.60 (m, 2H), 2.05 (s, 3H), 2.60-3.20 (m, 8H), 3.35 (s, 4H), 3.65 (t, 2H), 3.80 (s, 3H), 6.85 (d, 2H), 7.25 (t, 1 H).

Analysis, theoretical (with 1/2 H₂0): C, 64.93; H, 7.94; N, 6.58. Found: C, 64.62; H, 7.79; N, 6.52.

### Example 24

### Preparation of 4-Methoxy-2-(4-(2-phthalimido)butyl)-1,2,3,3a,8,8a-hexahydroindeno[1,2-c]pyrrole hydrochloride

The product from Example 6 (0.60 g) was combined with 0.84 g. 1-bromo-4-(phthalimidyl)-butane and 1.4 ml diisopropyl ethyl amine in 10 ml acetonitrile. After 18 h at reflux, the reaction was quenched in aqueous 5% NaHCO₃ solution and extracted with diethyl ether. The organic layer was washed with brine, dried (K₂CO₃) and evaporated. The resulting oil was treated with ethereal HCI, and the resulting hydrochloride salt was recrystallized from EtOAc/ether to yield 0.56 g of a white solid, mp: 196-198° C. NMR (d₆ DMSO) δ 1.65 (m, 4H), 2.60-3.20 (m, 6H), 3.60 (m, 2H), 3.80 (s, 3H), 3.90 (m, 2H), 6.80 (m, 2H), 7.20 (m, 1H), 7.75 (m, 4H)

Analysis, theoretical: C, 67.52; H, 6.37; N, 6.56. Found: C, 67.39; H, 6.47; N, 6.46.

### Example 25

### Preparation of 4-Hydroxy-2-(4-(2-phthalimido)butyl)-1,2,3,3a,8,8a-hexahydroindeno[1,2-c]pyrrole hydrobromide

The resultant of Example 24 (0.53 g) was treated as described in Example 10 to yield 0.47 g of the desired product, mp: 185-190° C NMR (CDCl₃) δ 1.20 (t, 1 H), 1.25-2.00 (m, 5H), 2.50-3.20 (m, 6H), 3.50 (q, 1 H), 3.60-4.50 (m, 5H), 6.75 (d, IH), 6.90 (d, 1 H) 7.10 (t, 1 H), 7.60-7.90 (m, 4H).

Analysis, theoretical (with 1/2 H₂O) : C, 59.23; H, 5.62; N, 6.01; Found: C, 58.88; H, 5.55; N, 5.53.

### Example 26

### Preparation of 2-(3-(3,3-tetramethylene)glutarimidyl)propyl-4-methoxy-1,2,3,3a,8,8a-hexahydroindeno[1,2-c]pyrrole hydrochloride

The resultant of Example 6 (1.00 g) was dissolved in 20 ml of acetonitrile and 2.3 ml diisopropyl ethyl amine. To the reaction was added 1.40 g 1-bromo-3-(3,3-tetramethyleneglutarimidyl)-propane, and the reaction was heated at reflux for 18 h The reaction was quenched in aqueous 5% NaHCO₃ solution, extracted with ether dried (K₂CO₃) and evaporated. The resulting oil was treated with ethereal HCI, and the resulting hydrochloride salt was recrystallized from methylene chloride/ether to yield 0.53 g of a white solid, mp: 170-174° C. NMR (CDCl₃) 8 1.50 (m, 4H), 1.70 (m, 4H), 2.10 (m, 2H), 2.40 (q, 1H), 2.60-3.00 (m, 4H), 3.20 (q, 1H), 3.60 (m, 1H), 3.80-3.90 (m, 6H), 4.10-4.30 (m, 3H), 6.80 (q, 2H), 7.25 (t, 1 H).

Analysis, theoretical (with 1/4 H₂0): C, 65.89; H, 7.60; N, 6.40. Found: C, 65.80; H, 7.76; N, 6.24.

### Example 27

### Preparation of 2-(3-(3,3-tetramethylene)glutarimidyl)propyl-4-hydroxy-1,2,3,3a,8,8a-hexahydroindeno[1,2-c]pyrrole hydrobromide

The product from Example 26 (0.30 g) was treated as described in Example 10 to yield 0.27 g of the desired product, mp: 152-6°C. NMR (CDCl₃) 8 1.20 (t, 1 H), 1.50 (m, 4H), 1.70 (m, 4H), 2.10 (m, 2H), 2.60 (s, 4H), 2.80 (d, 1 H), 3.00 (m, 3H), 3.20 (q, 1 H), 3.50 (q, 1 H), 3.80 (t, 2H), 3.90 (m, 1 H), 4.20 (m, 1 H), 4.40 (m, 1 H), 6.70-6.90 (q, 2H), 7.10 (1, 3H).

Analysis, theoretical (with 1/2 H₂0): C, 58.47; H, 6.83; N, 5.93. Found: C, 58.48; H, 6.86; N, 5.79.

### Example 28

### Preparation of 2-(5-(3,3-tetramethylene)glutarimidyl)pentyl-4-methoxy-1,2,3,3a,8,8a-hexahydroindeno[1,2-c]pyrrole hydrochloride

The product from Example 6 (1.05 g) was combined with 1.66 g. 1-bromo-5-(3,3-tetramethyleneglutarimidyl) - pentane and 2.4 ml diisopropyl ethyl amine in 50 ml acetonitrile. After 18 h at reflux, the reaction was quenched in aqueous 5% NaHC0₃ solution and extracted with diethyl ether. The organic layer was washed with brine, dried (K₂CO₃) and evaporated. The resulting oil was treated with ethereal HCl, and the resulting hydrochloride salt was lyophilized to yield a white amorphous solid. NMR (CDCl₃) 8 1.30-1.90 (m, 15H), 2.40 (m, 1 H), 2.60 (s, 1 H), 2.70-2.90 (m, 4H). 3.20 (s, 3H), 3.60 (m, 1 H), 3.70 (t, 2H), 3.80 (s, 3H), 4.10-4.30 (m, 2H), 6.70 (d, 1 H), 6.85 (d, 1 H), 7.25 (t, 1 H). Analysis, theoretical (with 2 moles H₂O) : C, 62.82; H, 8.31; N, 5.64. Found: C, 62.79; H, 7.76; N, 5.48.

### Example 29

### Preparation of 2-(4-(3,3-tetramethylene)glutarimidyl)-cis-but-2-enyl-4-methoxy-1,2,3,3a,8,8a-hexahydroindeno [1,2-c] pyrrole hydrochloride

The product from Example 6 (1.05 g) was treated with 1.25 g cis-1-bromo-4-(3,3-tetramethyleneglutarimidyl)-2-butene as described in Example 28 to yield 0.75 g of a white amorphous solid. NMR (CDCl₃) δ 1.50 (m, 4H), 1.70 (m, 4H), 2.55 (s, 4H), 2.60-3.60 (m, 6H), 3.80 (m, 5H), 4.10-4.30 (m, 4H), 5.70-6.00 (m, 2H), 6.75-6.85 (q, 2H), 7.25 (t, 1 H).

Analysis, theoretical (with 1/4 H₂0): C, 66.80; H, 7.51; N, 6.23. Found: C, 66.50; H, 7.44; N, 6.18.

### Example 30

### Preparation of 2-(4-(3,3-tetramethylene)glutarimidyl)-trans-but-2-enyl-4-methoxy-1,2,3,3a,8,8a-hexahydroindeno

### [1,2-c]pyrrole hydrochloride

The product from Example (0.80 g) was combined with 1.17 g trans-1-bromo-4-(3,3-tetramethylene-glutarimidyl)-2-butene and 1.8 ml diisopropyl ethyl amine in 50 ml acetonitrile. After 18h at reflux, the reaction was quenched in 5% NaHC0₃ and extracted with ether. The organic layer was washed with brine, dried (K₂CO₃) and evaporated. The resulting oil was treated with ethereal HCI, and the resulting hydrochloride salt was recrystallized from methylene chloride/ether to yield 0.46 g of a white solid, mp: 155-157° C. NMR (CDCI₃) δ 1.50 (m, 4H), 1.70 (m, 4H), 2.40 (q, 1H), 2.70 (s, 4H), 2.80-3.75 (m, 8H), 4.10 (m, 2H), 4.40 (m, 2H), 5.70-5.90 (m, 2H), 6.70-6.80 (q, 2H), 7.25 (t, 1 H).

Analytical, theoretical (with 1/4 H₂0) : C, 66.79; H, 7.40; N, 6.23. Found: C, 67.05; H, 7.51; N, 6.06.

### Example 31

### Preparation of 2S-(1,4-benzodioxanyl-2-methyl)-4-methoxy-1,2,3,3a,8,8a-hexahydroindeno[1,2-c]pyrrole hydrochloride

The product from Example 6 (0.300 g) was dissolved in 10 ml acetonitrile and 1.0 ml diisopropyl ethyl amine. To the solution was added 0.600 g R-2-tosyloxymethyl-1,4-benzodioxan, and the reaction was heated at reflux for 18 h. The product, after chromatographic purification, was converted to its hydrochloride salt, mp: 196-8° C. NMR (d₆ DMSO) 8 2.70-3.95 (m, 10H), 3.80 (s, 3H), 4.05 (m, 1H), 4.27 (m, 1H), 4.75 (m, 1 H), 6.88 (m, 6H), 7.23 (m, 1 H).

Analysis, theoretical (1/2.H₂0): C, 65.88; H, 6.58; N, 3.66. Found: C, 65.40; H, 6.44; N, 3.54.

### Example 32

### Preparation of 2R-(1,4-benzodioxanyl-2-methyl)-4-methoxy-1,2,3,3a,8,8a-hexahydroindeno[1,2-c]pyrrole hydrochloride

The product from Example 6 (0.300 g) was treated with 0.600 g 2-S-tosyloxymethyl-1,4-benzodioxan as described in Example 31 to yield 0.340 g of the desired product as a white solid, mp: 190-2° C. NMR (d₆ DMSO) 8 2.70-3.70 (m, 8H), 3.80 (s, 3H), 3.88-4.18 (m, 3H), 4.30 (m, 1 H), 4.79 (m, 1 H), 6.80-7.50 (m, 7H).

Analysis, theoretical (1/2.H₂0): C, 65.88; H, 6.58; N, 3.66. Found: C, 65.27; H, 6.33; N, 3.35.

### Example 33

### Preparation of 4-methoxy-2-(2-phenyl)ethyl-1,2,3,3a,8,8a-hexahydroindeno[1,2-c]pyrrole hydrochloride

The product from Example 6 (0.870 g) was converted to its free base and treated with 0.654 g phenylacetic acid and 0.912 g dicyclohexylcarbodiimide in tetrahydrofuran. The resulting amide was treated with 0.900 g LiAIH₄ in 30 ml tetrahydrofuran at 25° C. for 3h, quenched by the addition of H₂O and 15% NaOH, and then converted to its HCl salt to yield 0.570 g of a white solid, mp: 186-8⁰ C, NMR (d₆ DMSO) δ 2.70-3.20 (m, 9H), 3.80 (s, 3H), 3.81-4.11 (m, 3H), 6.83 (m, 2H), 7.29 (m, 6H).

Analysis, theoretical (1/4.H₂⁰): C, 71.84; H, 7.39; N, 4.19. Found: C, 72.19; H, 7.24; N, 4.11.

### Example 34

### Preparation of 4-Bromo-7-methoxy-2,3-dihydro-1H-inden-1-one

Polyphosphoric acid (600 g) was heated to 90° C and 30 g 2-bromo-5-methoxyphenyl propionic acid was added over 25 min. The reaction was stirred an additional 10 min and then quenched by addition to 2 kg ice The reaction mixture was extracted with methylene chloride, and the organic extracts were washed with 5% NaHCO₃, brine, dried (MgS0₄), and evaporated. The product was recrystallized from methanol to yield 15.10 g of a light yellow solid, mp: 125-8° C NMR (CDCl₃) δ 2.70 (m, 2H), 3.00 (m, 2H), 3.95 (s, 3H), 6.73 (d, 1 H), 7.66 (d, 1 H).

### Example 35

### Preparation of 4-Bromo-7-methoxy-1-oxo-2,3-dihydro-1H-indene-2-carboxaldehyde diethyl acetal

Triethyl orthoformate (16.5 ml) was cooled to -30° C and 15.0 ml BF₃·Et₂O in 50 ml methylene chloride was added. The reaction was warmed to 0° C for 15 min, and then cooled to - 78° C. To the reaction was added 12.05 g of the product from Example 34 in 50 ml methylene chloride, followed by dropwise addition of 19.4 ml diisopropyl ethyl amine. The reaction was then warmed to -15° C. for 2h, then quenched in 5% NaHCO₃ solution and extracted with methylene chloride. The organic extracts were washed with brine, dried (MgSO₄), and the solvent removed. The crude product was triturated with cold hexane, and the crystalline product collected to yield 14.60 g of a white solid, mp: 76-8° C. NMR (CDCl₃) δ 1.05 (t, 3H), 1.27 (t, 3H), 3.03 (m, 2H), 3.30 (m, 1 H), 3.43-3.80 (m, 4H), 3.95 (s, 3H), 5.00 (d, 1 H), 6.71 (d, 1 H), 7.63 (d, 1 H).

### Example 36

### Preparation of 4-Bromo-7-Methoxy-2,3-dihydro-1H-1-carboethoxymethylene-2-carboxaldehyde, diethyl acetal

Ethyl trimethylsilyl acetate (1.12 g) was dissolved in 10 ml tetrahydrofuran and cooled to -78° C. To the solution was added 7.0 ml lithium hexamethyldisilazide (1.0 M in THF). After 20 min, 1.71 g of the product from Example 35 in 10 ml THF was added. The reaction was stirred at -78° C for 2.5 h and then warmed to 25° C, and then quenched in H₂0 and extracted with ether The organic extracts were washed with brine, dried (MgSO₄), and evaporated to yield 1.96 g of the desired product as a white solid, mp: 73-5° C. NMR (CDCl₃) d 0.85 (t, 3H), 1.37 (t, 3H), 1.42 (t, 3H), 2.90 (dd, 1 H), 3.27 (m, 2H), 3.52-3.81 (m, 4H), 3.90 (s, 3H), 4.21 (q, 2H), 4.78 (d, 1 H), 6.62 (d, IH), 6.90 (d, 1 H), 7.39 (d, 1 H).

### Example 37

### Preparation of 7-Methoxy-2,3-dihydro-1H-indene-2-carboxaldehyde, diethyl acetal-1-acetic acid, ethyl ester

The product from Example 36 (10.0 g) was dissolved in ethanol (250 ml) and 3.63 g NaOAc.H₂0 was added, followed by 2.0 g 10% Pd/C. The reaction was hydrogenated at 4 atm for 4h, filtered, and the solvent removed. The product was dissolved in ether, washed with 5% NaHCO₃ solution, dried (MgS0₄), and the solvent evaporated to yield 7.80 g of the desired product as a colorless oil. NMR (CDCl₃) 8 1.20 (m, 9H), 2.49 (dd, IH), 2.70 (dd, 1 H), 2.85 (m, 3H), 3.52 (q, 2H), 3.70 (m, 3H), 3.80 (s, 3H), 4.03 (q, 2H), 4.73 (d, 1 H), 6.67 (d, 1 H), 6.80 (d, 1 H), 7.13 (t, 1 H).

### Example 38

### Preparation of 7-Methoxy-23-dihydro-1 H-indene-2-carboxaldehyde-1-acetic acid, ethyl ester

The product from Example 37 (2.90 g) was dissolved in 40 ml THF and 8 ml 6N aq. HCI. After 45 min and 25° C., the reaction was quenched in 5% NaHC0₃ solution and extracted with ether. The ether extracts were dried (MgS0₄), and evaporated to dryness to yield 1.98 g of the desired product as a mixture of cis- and trans-isomers. NMR (CDCI₃) δ 1.75 (m, 3H), 2.48 (m, 1H), 2.90 (m, 1H), 3.15 (m, 2H), 3.70 (m, 1H), 3.80 (s, 3H), 4.15 (m, 3H), 6.68 (m, 1H), 6.82 (m, 1 H), 7.17 (m 1 H), 9.80 and 9.90 (two d, 1 H).

### Example 39

### Preparation of 7-Methoxy-2.3-dihydro-1 H-indene-2-carboxylic acid-1-acetic acid, diethyl ester

The product from Example 38 (2.09 g) was dissolved in 35 ml ethanol. To the solution was added 2.93 g AgNO₃ in 5 ml H₂0, followed by 2.40 g KOH in 35 ml H₂0. After 40 min., the reaction was filtered and the aqueous solution was extracted with ether The aqueous solution was acidified to pH 1 and extracted with methylene chloride; and the organic phase was washed with brine, dried (MgSO₄), and evaporated. The residue was dissolved in absolute ethanol and 0.5 ml 96% H₂SO₄ was added. The reaction was heated at reflux for 2h, quenched in 5% NaHCO₃, and extracted with ether. The organic extracts were dried (MgSO₄) and evaporated to yield 1 99 g of the desired product as a colorless oil. NMR (CDCl₃) δ 1.25 (m, 6H), 2.50 (m, 2H), 3.25 (m, 4H), 3.80 (s, 3H), 4.12 (m, 4H), 6.67 (d, 1 H), 6.80 (d, 1 H), 7.17 (t, 1 H).

### Example 40

### Preparation of cis- and trans-2-benzyl-5-methoxy-2,3,4,4a,9,9a-hexahydro-1H-indeno[2,1-c]pyridine-1,3-dione

Benzyl amine (4.82 g) was dissolved in 60 ml toluene. To the solution was added 22.5 ml of 2.0 M Me₃AI in toluene. After 1h at 25° C., 4.59 g of the product from Example 39 in 25 ml toluene was added. The reaction was heated at reflux for 3h, and then cooled to 0° C, and 10 ml H₂0 was added dropwise. The reaction was then poured into dilute HCl solution and extracted with ethyl acetate. The organic extracts were dried (MgSO₄), and evaporated. The resulting product was suspended in 300 ml xylene and 3.14 g pTsOH·H₂O. The reaction was stirred at reflux for 18 h, and then evaporated to dryness. The resulting product was purified by chromatography to yield 1.03 g of the trans-isomer, NMR (CDCl₃) δ 2.70 (m, 2H), 3.05 (dd, 1 H), 3.17 (dd, 1 H), 3.37 (dt, 1 H), 3.75 (dd, 1 H), 3.80 (s, 3H), 4.98 (dd, 2H), 6.72 (d, 1 H), 6.91 (d, 1 H), 7.20 (t, 1 H), 7.22-7.40 (m, 5H). Further elution yielded 2.38 g of the cis-isomer, NMR (CDCl₃) δ 2.83 (dd, 1 H), 3.03 (dd, 1 H), 3.24 (dd, 1 H), 3.43 (m, 2H), 3.75 (m, 1 H), 3.81 (s, 3H), 4.96 (s, 2H), 6.71 (d, 1 H), 6.84 (d, 1 H), 7.20 (m, 6H).

### Example 41

### Preparation of cis-2-benzyl-5-methoxy-2,3,4,4a,9,9a-hexahydro-1H-indeno[2,1-c]pyridine hydrochloride

The cis-isomer from Example 40 (2.35 g) was dissolved in 100 ml tetrahydrofuran. To the solution was added 2.25 g LiAIH₄. The reaction was stirred at 25° C. for 3 h, and then quenched by the addition of 2.35 ml H₂0, 2.35 ml 15% NaOH, and 7.0 ml H₂0, filtered and the solvent evaporated. The resulting product was treated with ethereal HCl to yield 2.26 g of the desired product as a white solid, mp: 216-8° C. NMR (d₆ DMSO) δ 1.70 (m, 1 H), 2.00-3.55 (m, 8H), 3.78 (s, 3H), 4.18-4.44 (m, 3H), 6,85 (m,m 2H), 7.16 (m, 1H), 7.40 (m, 3H), 7.58 (m, IH), 7.70 (m, 1H).

### Example 42

### Preparation of cis-5-methoxy-2,3,4,4a,9,9a-hexahydro-1H-indeno[2,1-c]pyridine hycrochloride

The product from Example 41 (1.80 g) was combined with 0.90 g 20% Pd/C in 100 ml methanol and hydrogenated at 4 atm for 4 h. The reaction was filtered and evaporated to yield 1.08 g of the desired product as a white solid, mp: 140-3° C. NMR (d₆ DMSO) δ 1.70 (m, 1 H), 2.02 (m, 1 H), 2.60 (m, 1 H), 2.78-3.30 (m, 7H), 3.77 (s, 3H), 6.80 (d, 1 H), 6.84 (d, 1 H), 7.15 (t, 1 H).

### Example 43

### Preparation of cis-2-(3-(3,3-tetramethylene)glutarimidyl)propyl-5-methoxy-2,3,4,4a,9,9a-hexahydro-1H-indeno[2,1-c] pyridine hydrochloride

The product from Example 42 (0.479 g) was treated as described in Example 26 to yield the desired product as a white solid, mp: 170-2° C. NMR (d₆ DMSO) δ 1.40 (m, 4H), 1.62 (m, 4H), 1.80-3.70 (m, 16H), 2.62 (s, 4H), 3.78 (s, 3H), 6.78 (d, 1 H), 6.83 (d, 1 H), 7.15 (t, 1 H).

Analysis, theoretical (1/4.H₂0) : C, 66.50; H, 7.92; N, 6.20. Found: C, 66.48; H, 7.76; N, 6.12.

### Example 44

### Preparation of cis-2-(4-(3,3-tetramethylene)glutarimidyl)propyl-5-methoxy-2,3,4,4a,9,9a-hexahydro-1H-indeno[2,1-c] pyridine hydrochloride

The product from Example 42 (0.479 g) was treated as described in Example 7 to yield, after lyophilization, the desired product as a non-crystalline amorphous powder. NMR (d₆ DMSO) 8 1.40 (m, 4H), 1.65 (m, 6H), 1.90-3.70 (m, 16H), 2.62 (s, 4H), 3.78 (s, 3H), 6.78 (d, 1H), 6.87 (d, 1H), 7.17 (t, 1H).

Analysis, theoretical (1·H₂O): C, 65.18; H, 8.20; N, 5.85. Found: C, 65.65; H, 7.96; N, 5.83.

### Example 45

### Preparation of cis-5-methoxy-2-propyl-2,3,4,4a,9,9a-hexahydro-1H-indeno[2,1-c]pyridine hydrochloride

The product from Example 42 (0.546 g) was treated as described in Example 9 to yield 0.428 g of the desired product as a white solid, mp: 175-7° C. NMR (d₆ DMSO) δ 0.90 (t, 3H), 1.20 (m, 4H), 1.95-3.55 (m, 10H), 3.78 (s, 3H), 6.78 (d, 1H), 6.84 (d, 1H), 7.15 (t, 1H).

Analysis, theoretical: C, 68.19; H, 8.58; N, 4.97. Found: C, 68.01; H, 8.55; N, 4.93.

### Example 46

### Preparation of cis-5-hydroxy-2-propyl-2,3,4,4a,9,9a-hexahydro-1 H-indeno[2,1-c]pyridine hydrobromide

The product from Example 45 (0.563 g) was treated as described in Example 10 to yield 0.247 g of the desired product as a white solid, mp: 204-6° C. NMR (d₆ DMSO) δ 0.90 (t, 3H), 1.70 (m, 4H), 2.05-3.55 (m, 10H), 6.60 (d, 1H), 6.70 (d, 1H), 6.95 (t, 1 H).

Analysis, theoretical: C, 57.70; H, 7.10; N, 4.49. Found: C, 57.67; H, 7.12; N, 4.43.

### Example 47

### Preparation of trans-2-benzyl-5-methoxy-2,3,4,4a,9,9a-hexahydro-1 H-indeno[2,1-c]pyridine hydrochloride

The trans-isomer from Example 41 (1.00 g) was treated as described in Example 38 to yield 0.834 g of the desired product as a white solid, mp: >260° C. NMR (d₆ DMSO) δ 1.95 (m, 1 H), 2.28 (m, 1 H), 2.38-3.55 (m, 8H), 3.73 (s, 3H), 4.34 (dd, 2H), 6.80 (d, 1H), 6.84 (d, 1 H), 7.13 (t, 1 H), 7.48 (m, 3H), 7.62 (m, 2H).Preparation of trans-5-methoxy-2,3,4,4a,9,9a-hexahydro-1H-indeno[2,1-c]pyridine hydrochloride.

### Example 48

### Preparation of trans-5-Methoxy-2,3,4,4a,9,9a-hexahydro-1H-indeno[2,1-c]pyridine hydrochloride

The product from Example 47 (0.83 g) was treated as described in Example 42 to yield 0.57 g of the desired product as a white solid, mp: >260°C. NMR (d₆ DMSO) 8 1.60-2.10 (m, 4H), 2.55-3.55 (m, 6H), 3.74 (s, 3H), 6.79 (d, 1 H), 6.87 (d, 1 H), 7.13 (t, 1 H).

### Example 49

### Preparation of trans-5-methoxy-2-propyl-2,3,4,4a,9,9a-hexahydro-1H-indeno[2,1-c]pyridinehydrochloride

The product from Example 48 (0.118 g) was treated as described in Example 9 to yield 0.075 g of the desired product as a white solid, mp: >260° C. NMR (d₆ DMSO) δ 0.92 (t, 3H), 1.73 (m, 2H), 1.85-3.70 (m, 12H), 3.75 (s, 3H), 6.81 (d, 1 H), 6.87 (d, 1 H), 7.14 (t, 1 H).

Analysis, theoretical: C, 68.19; H, 8.54; N, 4.93. Found: C, 68.37; H, 8.54; N, 4.97.

### Example 50

### Preparation of trans-2-(3-(3,3-tetramethylene)glutarimidyl)propyl-5-methoxy-2,3,4,4a,9,9a-hexahydro-1 H-indeno [2,1-c]pyridine hydrochloride

The product from Example 48 (0.240 g) was treated as described in Example 26 to yield 0.204 g of the desired product as a white solid, mp: 221-5° C. NMR (d₆ DMSO) δ 1 43 (m, 4H), 1 55 (m, 4H), 1.90 (m, 4H), 2.20 (m, 2H), 2.62 (s, 4H), 2.30-3.25 (m, 7H), 3.52 (m, IH), 3.70 (t, 2H), 3.77 (s, 3H), 6.80 (d, 1H), 6.88 (d, 1H), 7.13 (t, 1 H).

Analysis, theoretical (1/4.H₂0): C, 66.50; H, 7.92; N, 6.20. Found: C, 66.39; H, 7.79; N, 6.13.

### Example 51

### Preparation of trans-2-(4-(3,3-tetramethylene)glutarimidyl)butyl-5-methoxy-2,3,4,4a,9,9a-hexahydro-1H-indeno

### [2,1-c] pyridine hydrochloride

The product from Example 48 (0.300 g) was treated as described in Example 7 to yield 0.310 g of the desired product as a white solid, mp: 222-4° C. NMR (d₆ DMSO) 8 1.40 (m, 6H), 1.62 (m, 6H), 2.10-3.60 (m, 12H), 2.62 (s, 4H), 3.66 (m, 2H), 3.74 (s, 3H), 6.80 (d, 1 H), 6.87 (d, 1 H), 7.13 (t, 1 H).

Analysis, theoretical: C, 67.73; H, 8.09; N, 6.08. Found: C, 67.43; H, 8.02; N, 5.95.

### Example 52

### Preparation of 2-(4-(3,3-Tetramethylene)glutarimidyl)butyl-5-chloro-4-hydroxy-1,2,3,3a,8,8a-hexahydroindeno[1,2-c]-pyrrole hydrobromide

The product from Example 8 (0.400 g) was treated as described in Example 10 to yield 0.407 g of the desired product as a white solid, mp: 171-4° C. NMR (d₆ DMSO) 8 1.40 (m, 4H), 1.62 (m, 6H), 2.62 (s, 4H), 2.70 to 4.10 (m, 14H), 6.70 (d, 1 H), 7.13 (d, 1 H).

Analysis, theoretical (1/2 H₂0): C, 55.34; H, 6.39; N, 5.38. Found: C, 55.52; H, 6.19; N, 5.27.

### Example 53

### Preparation of 4-Methoxy-2(4-(4-morpholin-3,5-dionyl)butyl)-1,2,3,3a,8,8a-hexahydroindeno-[1,2-c]pyrrole hydrochlorid

The product from Example 6 (0.800 g) was condensed with 1-bromo-4(4-morpholin-3,5-dionyl)butane (1.05 g) as described in Example 7 to yield 0.725 g of the desired product as a white solid, mp: 160-2° C. NMR (d₆ DMSO) δ 1.65 (m, 2H), 1.92 (m, 2H), 2.42 (m, 1 H), 2.70 to 3.10 (m, 4H), 3.20 (m, 1 H), 3.55 (m, 1 H), 3.70 to 4.40 (m, 5H), 3.80 (s, 3H), 4.40 (s, 4H), 6.72 (d, 1 H), 6.83 (d, 1 H), 7.24 (t, 1 H).

Analysis, theoretical (1/4 H₂0): C, 60.15; H, 6.94; N, 7.01. Found: C, 60.27; H, 6.87; N, 7.02.

### Example 54

### Preparation of 4-Methoxy-2-(4-(3a,4,4a,6a,7,7a-hexahydro-4,7-etheno-1H-cyclobut[flisoindol-1,3-dionyl)-butyl)-1,2,3,3a,8,8a-hexahydroindeno-[1,2-c]pyrrole hydrochloride

The product from Example 6 (0.451 g) was condensed with 0.81 g 4-bromo-1-(3a,4,4a,6a,7,7a-hexahydro-4,7-etheno-1 H-cyclobut[f]isoindol-1,3-dionyl)-butane as described in Example 7 to yield 0.380 g of the desired product as a white solid, mp: 170-2° C. NMR (d₆ DMSO) δ 1.48 (m, 4H), 2.40 to 4.10 (m, 21 H), 3.79 (s, 3H), 5.82 (m, 2H), 5.90 (s, 2H), 6.83 (m, 2H), 7.22 (t, 1 H).

Analysis, theoretical (2 H₂0): C, 65.01; H, 7.21; N, 5.42. Found: C, 64.85; H, 6.72; N, 5.35.

### Example 55

### Preparation of 4-Methoxy-2-(4-(3aα,4a,5,6,7aα-hexahydro-4,7-methano-1H-isoindol-1,3(2H)-dionyl)-butyl)-1,2,3,3a, 8,8a-hexahydroindeno[1,2-c]pyrrole hydrochloride

The product from Example 6 (0.350 g) was condensed with 0.65 g 1-bromo-4-(3aa,4a,5,6,7a,7a a-hexahydro-4,7-methano-1 H-isoindol-1,3,(2H)-dionyl)-butane as described in Example 7 to yield 0.410 g of the desired product as a white solid, mp: 164-6° C NMR (CDCl₃) δ 1.18 (m, 2H), 1.60 (m, 6H), 1.92 (m, 2H), 2.42 (q, 1 H), 2.70 to 3.95 (m, 13H), 3.82 (s, 3H), 4.20 (m, 2H), 6.72 (d, 1 H), 6.85 (d, 1 H), 7.23 (t, 1 H).

Analysis, theoretical (1/2 H₂0): C, 66.14; H, 7.55; N, 6.17. Found: C, 66.14; H, 7.25; N, 6.10.

### Example 56

### Preparation of trans-2(3-(2-(1,2-benzisothiazolin-3-one-1,1-dioxyde))propyl)-5-methoxy-2,3,4,4a,9,9a-hexahydro-1 H-indeno[2,1-c]pyridine hydrochloride

The product from Example 48 (0.480 g) was treated with 0.730 g 3-bromopropyl-2-(1,2-benzisothiazolin-3-one-1,1-dioxide as described in Example 7 to yield 0.463 g of the desired product as a white solid, mp: 252-3° C. NMR (dₛ DMSO) δ 1.90 (m, 2H), 2.22 (m, 4H), 2.58 (m, 2H), 2.80 (m, 2H), 2.95 - 3.45 (m, 4H), 3.60 (m, 2H), 3.74 (s, 3H), 3.86 (t, 2H), 6.80 (d, 1 H), 6.87 (d, 1 H), 7.12 (t, 1 H), 8.08 (m, 3H), 8.34 (d, 1 H).

Analysis theoretical (1/2 H₂O): C, 58.53; H, 5.98; N, 5.94. Found: C, 58.03; H, 5.82; N, 6.11.

### Example 57

### Preparation of trans-2(4-(2-(1,2-benzisothiazolin-3-one-1,1-dioxyde))butyl)-5-methoxy-2,3,4,4a,9,9a-hexahydro-1H-indeno[2,1-c]pyridine hydrochloride

The product from Example 48 (0.330 g) was treated with 0.48 g 4-bromobutyl-2-(1,2-benzisothiazolin-3-one-1,1-dioxide as described as described in Example 7 to yield 0.363 g of the desired product as a white solid, mp: 228-30° C. NMR (dₛ DMSO) δ 1.90 (m, 4H), 2.22 (m, 4H), 2.58 (m, 2H), 2.80 (m, 2H), 2.95 - 3.45 (m, 4H), 3.60 (m, 2H), 3.74 (s, 3H), 3.86 (t, 2H), 6.80 (d, 1 H), 6.87 (d, 1H), 7.12 (t, 1 H), 8.08 (m, 3H), 8.34 (d, 1 H).

Analysis theoretical: C, 60.43; H, 6.13; N, 5.78. Found: C, 60.11; H, 6.07; N, 5.78.

### Example 58

### Preparation of trans-2-(2-(4-fluorobenzamido)ethyl)-5-methoxy-2,3,4,4a,9.9a-hexahydro-1H-indeno[2,1-c]pyridine hydrochloride

The product from Example 48 (0.310 g) was treated with 0.34 g 2-bromo-1-(4-fluorobenzamido)ethane as described as described in Example 7 to yield 0.170 g of the desired product as a white solid, mp: 242-4° C. NMR (dₛ DMSO) 8 1.95 (m, 2H), 2.29 (m, 2H), 2.45 - 2.75 (m, 2H), 2.82 (m, 2H), 3.05 - 3.45 (m, 4H), 3.60 - 3.90 (m, 2H), 3.77 (s, 3H), 6.80 (d, 1 H), 6.87 (d, 1 H), 7.13 (t, 1 H), 7.31 (m, 2H), 8.00 (m, 2H), 8.98 (m, 1 H).

Analysis theoretical (1/4 H₂O): C, 64.54; H, 6.52; N, 6.84. Found: C, 64.48; H, 6.52; N, 6.81.

The 5-HT selectivity of the compounds of this invention were demonstrated bytesting the compounds in radioligand binding assays. The therapeutic activity of the compounds was demonstrated in vivo by the ability of the compounds to affect arterial blood pressure and/or heart rate in various experimental animals such as the spontaneously hypertensive (SH) rat.

Tissue preparation for the radioligand binding assays was performed as described by Pedigo, Yamamura, and Nelson, J. Neurochemistry, 36(1): 220-226 (1980). Male Sprague-Dawley rats (150-200 g) were sacrificed and the brains were rapidly removed and placed on ice. The frontal cortext, including the portion immediately above the striatum and part of the parietal cortex were dissected. Tissues from 6-10 rats were pooled and homogenized in at least 40 volumes of tris-HCI buffer (0.05 M, pH 7.4) using a Brinkman Polytron (setting 5 for 20 seconds). This homogenate was centrifuged (48,000 G for 10 minutes), the pellet resuspended, and the process repeated three more times. Between the second and third washes, the tissue homogenate was incubated for 10 minutes at 37°C. The final pellet was resuspended in 100 volumes of TRIS buffer for use in the binding assay.

Radioligand binding affinity for subtypes of the serotonin receptor was determined by the method of Pedigo, Yamamura, and Nelson, op. cit. Tissue homogenate (0.5 mL) from rat frontal cortex, together with various concentrations of tritiated 5-HT and test compounds were added to glass tubes containing modified tris-HCI buffer to obtain, in each case, a final volume of 2 mL and having the following compositions: 5.7 mM ascorbate, 10 micromolar pargyline, 4mM CaCl₂, 50 mM TRIS buffer; pH 7.4 at 37°C.

Each mixture was incubated for 15 minutes at 37°C and then vacuum filtered through Whatman GF/B filters followed by three 5 mL washes with cold TRIS buffer. Radioactivity was extracted overnight in 3 mL of scintillation liquid and measured by liquid scintillation spectrometry (50% efficiency). In all experiments, tritiated 5-HT binding was defined as the difference between binding in the absence and in the presence of 10 micromolar unlabeled 5-HT and represented 60-80% of the total radioactivity bound.

Binding parameters for saturation and drug displacement studies were calculated by Scatchard analysis and from Hill plots, respectively. Statistical comparisons of data fitted to a one-site versus a two-site model were made using the method of Hancock, et al., Mol. Pharmacol., 16: 1-9 (1979). The high affinity sites were determined to be identical to the 5-HT_{1A} site by conducting the binding assays in the presence of 1 micromolar siproxatriene, a selective 5-HT_{1A} ligand.

In another study, a group of spontaneously hypertensive male rats were trained to be restrained in a wire mesh cylinder in a warming box, at least two training cycles being conducted before testing. The rats were warmed for about one-hour prior to blood pressure measurement, the warming box being maintained at a constant temperature of 36°C. An occluding cuff attached to a programmed sphygmomanometer was placed near the base of the tail of each rat and the pressure of the cuff was increased automatically from 0 to 250 millimeters of mercury at a rate of 10 mm Hg pressure per second. The total time for each cycle of inflation and deflation of the cuff was 50 seconds and the interval between cycles was one minute A photocell, placed distally to the cuff, recorded the pulses due to the forward motion of blood flow with each heart beat. As the pressure in the cuff increased, the pulse disappeared completely at the point where the cuff pressure equalled and exceeded the arterial blood pressure. The pulse reappeared during deflation at approximately the same pressure. Five interference free signals were recorded for each rat. Rats with a blood pressure of 180 mm Hg or more during the control period were used in the study.

Blood pressure and heart rate readings were recorded on a Model VII Grass polygraph at various intervals after administration of the test compound. Table 1 shows structure-activity relationships of the compounds of the present invention and their radioligand binding (RLB) ability at the 5-HT, and 5-HT₂ receptors. The "high" and "low" affinity sites refer to the 5-HT_{1A} and 5-HT, _{B} subtyes of the serotonin receptors. For the in vivo test data, SHR refers to spontaneously hypertensive rats, dosed in milligrams per kilogram (mpk), with the data being expressed in the Table in +% changes at a particular dosage range. That is to say, "-17.5% at 30" represents a 17.5% decrease in blood pressure at a dose level of 30 mg/kg of body weight.

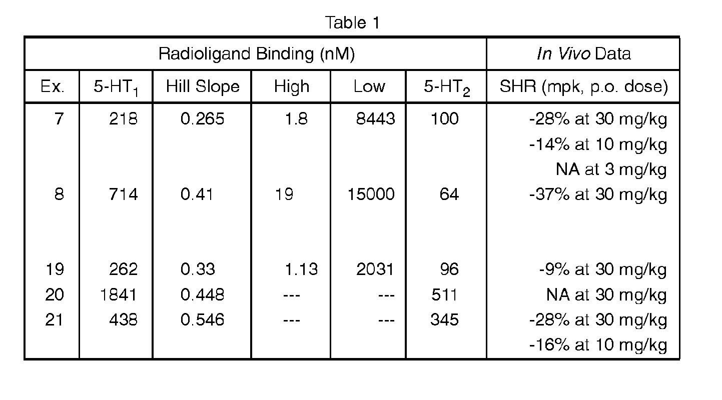

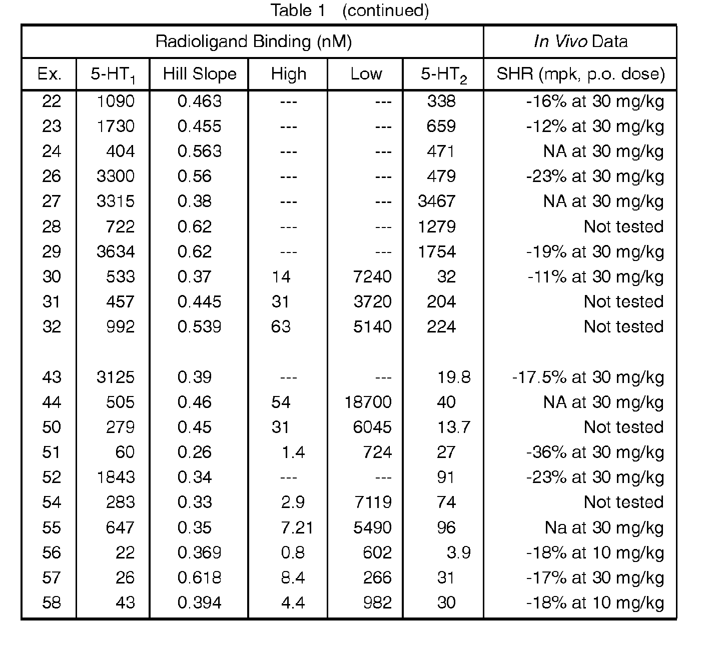

## Claims (Claims for the following Contracting State(s) : AT, BE, CH, DE, FR, GB, IT, LI, LU, NL, SE)

1. A compound of the formula 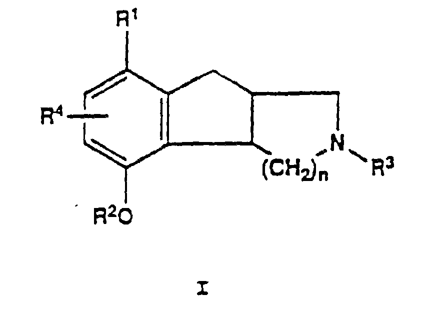
wherein R¹ is hydrogen or an electron withdrawing group
R² is hydrogen, lower alkyl, or arylalkyl;
n is an integer having a value of from 1 to 3;
R⁴ is a member selected from the group consisting of
hydrogen,
halogen,
lower alkyl,
lower alkoxy, and
aryl(lower alkyl); or
R²0 and R⁴ taken together form an alkylenedioxy bridge;
R³ is a member selected from the group consisting of
arylamidoalkenylene,
arylamidoalkylene,
aryl(lower alkyl)amidoalkenylene,
aryl(lower alkyl)amidoalkylene,
benzoalkylenedioxyalkylene,
a) a group of the formula: 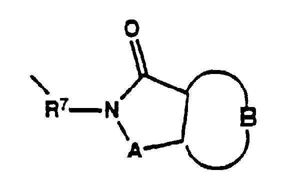
wherein B is benzo, cyclohexyl, or a bicyclo ring;
A is CO, SO, or S0₂, and
R⁷ is a bivalent aliphatic hydrocarbon;
b) a group of the formula: 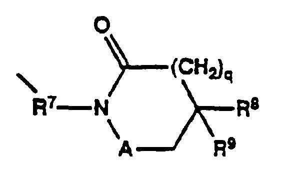 wherein q is an integer of from 0 to 3, R⁸ and R⁹ are hydrogen or lower alkyl, or R⁸ and R⁹ taken together form a ring of from 5 to 7 members and A and R⁷ are as defined above;
c) a group of the formula: 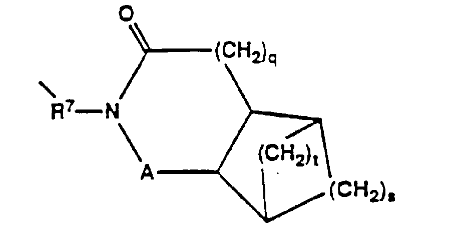 wherein s and t are independent integers of from 1 to 3, and q, A, and R⁷ are as defined above; and
d) a group of the formula 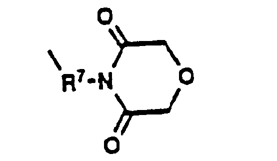 wherein R⁷ is as defined above;
wherein aryl refers to mono- or polycyclichydrocarbon fused or nonfused aromatic ring systems which may contain one or more hetero atoms in the ring system; and
wherein aryl wherever it appears and ring systems in the R³ group may be optionally substituted with halogen, lower alkyl, mono- or di(lower alkyl)- substituted lower alkyl, (lower alkyl)thio, halo-substituted lower alkyl, amino-substituted lower alkyl, mono- or di(lower alkyl)-substituted amino, lower alkenyl, lower alkynyl, lower alkoxy, aryloxy, aryl(lower alkyl), hydroxy, cyano, amino, mono- and di(lower alkyl)amino, or nitro; and
the pharmaceutically acceptable salts thereof.
Wherein the term "lower alkyl" refers to branched or unbranched saturated hydrocarbon radicals having from one to six carbon atoms.

2. A compound as defined by Claim 1 which is represented by the formula 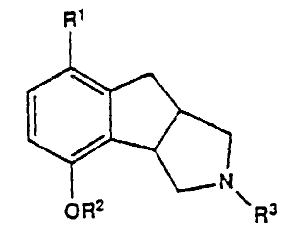 wherein R¹, R², and R³ are as defined therein.

3. A compound as defined by Claim 1 represented by the formula 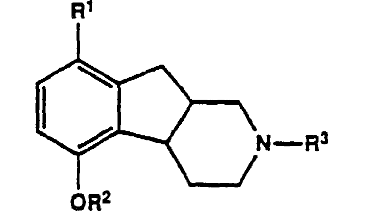 wherein R¹, R², and R³ are as defined therein.

4. A compound as defined by Claim 1 wherein R³ is selected from the group consisting of 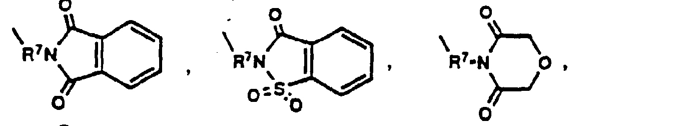 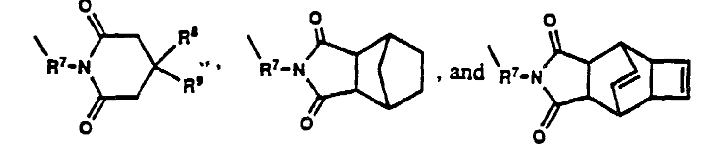 where R⁷, R⁸, and R⁹ are as defined therein.

5. A compound as defined by Claim 2 selected from the group consisting of
2R-(1,4-benzodioxanyl-2-methyl)-4-methoxy-1,2,3,3a,8,8a-hexahydroindeno[1,2-c]pyrrole;
2S-(1,4-benzodioxanyl-2-methyl)-4-methoxy-1,2,3,3a,8,8a-hexahydroindeno[1,2-c]pyrrole;
2-(3-(3,3-tetramethylene)glutarimidyl)propyl)-4-hydroxy-1,2,3,3a,8,8a-hexahydroindeno-[1,2-c]pyrrole;
2-(3-(3,3-tetramethylene)glutarimidyl)propyl)-4-methoxy-1,2,3,3a,8,8a-hexahydroindeno-[1,2-c]pyrrole;
2-(4-(3,3-tetramethylene)glutarimidyl)butyl-4-hydroxy-1,2,3,3a,8,8a-hexahydroindeno-[1,2-c]pyrrole;
2-(4-(3,3-dimethyl)glutarimidyl)butyl-4-methoxy-1,2,3,3a,8,8a-hexahydroindeno[1,2-c]pyrrole;
2-(4-(3,3-tetramethylene)glutarimidyl)butyl-4-methoxy-1,2,3,3a,8,8a-hexahydroindeno-[1,2-c]pyrrole;
2-(4-(3,3-tetramethylene)glutarimidyl)butyl-5-chloro-4-hydroxy-1,2,3,3a,8,8a-hexahydroindeno[1,2-c]pyrrole;
2-(4-(3,3-tetramethylene)glutarimidyl)butyl-5-chloro-4-methoxy-1,2,3,3a,8,8a-hexahydroindeno[1,2-c]pyrrole
2-(5-(3,3-tetramethylene)glutarimidyl)pentyl-4-methoxy-1,2,3,3a,8,8a-hexahydroindeno-[1,2-c]pyrrole;
2-(4-(3,3-tetramethylene)glutarimidyl)cis-buten-2-yl-4-methoxy-1,2,3;3a,8,8a-hexahydroindeno-[1,2-c]pyrrole
2-(4-(3,3-tetramethylene)glutarimidyl) trans-buten-2-yl-4-methoxy-1,2,3,3a,8,8a-hexahydroindeno-[1,2-c]pyrrole
4-hydroxy-2-(2-(4-Fluorobenzamido)ethyl)-1,2,3,3a,8,8a-hexahydroindeno[1,2-c]pyrrole;
4-methoxy-2-(2-(4-Fluorobenzamido)ethyl)-1,2,3,3a,8,8a-hexahydroinceno[1,2-c]pyrrole;
4-hydroxy-2-(4-(2-(1,2-Benzoisothiazolin-3-one-1,1-dioxide)butyl-1,2,3,3a,8,8a-hexahydroindeno [1,2-c]-pyrrole;
4-methoxy-2-(4-(2-(1,2-Benzoisothiazolin-3-one-1,1-dioxide))butyl)-1,2,3,3a,8,8a-hexahydroindeno[1,2-c]-pyrrole;
4-hydroxy-2-(4-(2-phthalimido)butyl)-1,2,3,3a,8,8a-hexahydroindeno-[1,2-c]pyrrole;and
4-methoxy-2-(4-(2-phthalimido)butyl)-1,2,3,3a,8,8a-hexahydroindeno-[1,2-c]pyrrole; and
pharmaceutically acceptable salts thereof.

6. A compound as defined by Claim 3 selected from the group consisting of
cis-2-(3,3-tetramethylene)glutarimidyl)propyl-5-methoxy-2,3,4,4a,9,9a-hexahydro-1H-indeno-[2,1-c]pyridine;
trans-2-(3,3-tetramethylene)glutarimidyl)propyl-5-methoxy-2,3,4,4a,9,9a-hexahydro-1H-indeno-[2,1-c]pyridine;
cis-2-(3,3-tetramethylene)glutarimidyl)butyl-5-methoxy-2,3,4,4a,9,9a-hexahydro-1H-indeno-[2,1-c]pyridine;
trans-2-(3,3-tetramethylene)glutarimidyl)butyl-5-methoxy-2,3,4,4a,9,9a-hexahydro-1H-indeno[2,1-c]pyridine;
trans-2(3-(2-(1,2-benzisothiazolin-3-one-1,1-dioxide))propyl)-5-methoxy-2,3,4,4a,9,9a-hexahydro-1H-indeno[2,1-c]pyridine;
trans-2(4-(2-(1,2-benzisothiazolin-3-one-1,1-dioxide))butyl)-5-methoxy-2,3,4,4a,9,9a-hexahydro-1H-indeno [2,1-c]pyridine; and
trans-2-(2-(4-fluorobenzamido)ethyl)-5-methoxy-2,3,4,4a,9,9a-hexahydro-1H-indeno-[2,1-c]pyridine hydrochloride; and
pharmaceutically acceptable salts thereof.

7. A compound of Claim 1 for use as a medicament.

8. A pharmaceutical composition comprising a compound as defined by Claim 1, or a pharmaceutically acceptable salt thereof, in combination with a pharmaceutically acceptable carrier.

9. Use of a compound of Claim 1 for manufacturing a medicament for modulating central 5-HT_{1A} receptors in a mammal in need of such treatment.

10. Use of a compound of Claim 1 for manufacturing a medicament for treating hypertension, anxiety or depression in a mammal in need of such treatment.

## Claims (Claims for the following Contracting State(s) : ES)

1. A process for preparing a compound of the formula 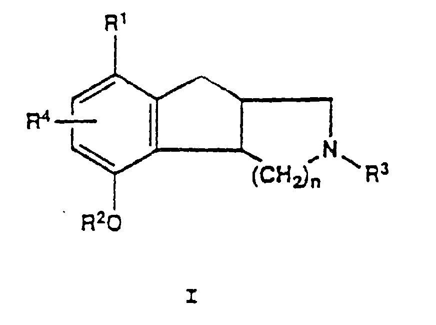
wherein R¹ is hydrogen or an electron withdrawing group
R² is hydrogen, lower alkyl, or arylalkyl;
n is an integer having a value of from 1 to 3;
R⁴ is a member selected from the group consisting of
hydrogen,
halogen,
lower alkyl,
lower alkoxy, and
aryl(lower alkyl); or
R²0 and R⁴ taken together form an alkylenedioxy bridge;
R³ is a member selected from the group consisting of
arylamidoalkenylene,
arylamidoalkylene,
aryl (lower alkyl) amidoalkenylene,
aryl(lower alkyl)amidoalkylene,
benzoalkylenedioxyalkylene,
a) a group of the fomula: 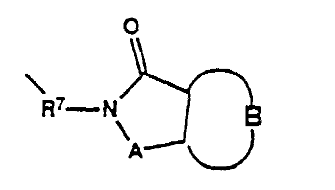
wherein B is benzo, cyclohexyl, or a bicyclo ring;
A is CO, SO, or SO₂, and
R⁷ is a bivalent aliphatic hydrocarbon;
b) a group of the formula: 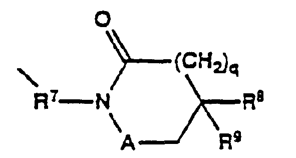 wherein q is an integer of from 0 to 3, R⁸ and R⁹ are hydrogen or lower alkyl, or R^{B} and R⁹ taken together form a ring of from 5 to 7 members and A and R⁷ are as defined above;
c) a group of the formula: 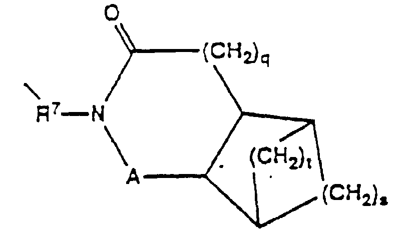 wherein s and t are independent integers of from 1 to 3, and q, A, and R⁷ are as defined above; and
d) a group of the formula 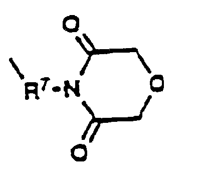
wherein R⁷ is as defined above;
wherein aryl refers to mono- or polycyclichydrocarbon fused or nonfused aromatic ring systems which may contain one or more hetero atoms in the ring system; and
wherein aryl wherever it appears and ring systems in the R³ group may be optionally substituted with halogen, lower alkyl, mono- or di(lower alkyl)- substituted lower alkyl, (lower alkyl)thio, halo-substituted lower alkyl, amino-substituted lower alkyl, mono- or di(lower alkyl)-substituted amino, lower alkenyl, lower alkynyl, lower alkoxy, aryloxy, aryl(lower alkyl), hydroxy, cyano, amino, mono- and di(lower alkyl)amino, or nitro; and
wherein the term "lower alkyl" refers to branched or unbranched saturated hydrocarbon radicals having from one to six carbon atoms; and
the pharmaceutically acceptable salts thereof; said process comprising reacting a compound of the formula 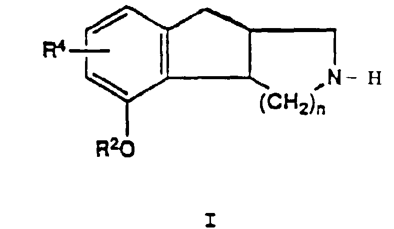 wherein R¹, R², R⁴ and n are as defined above, with a compound of the formula R³⁻halogen.

2. A process as defined by Claim 1 for preparing a compound represented by the formula 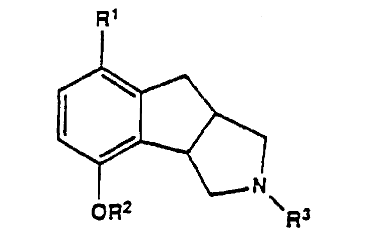 wherein R¹, R², and R³ are as defined therein

3. A process as defined by Claim 1 for preparing a compound represented by the formula 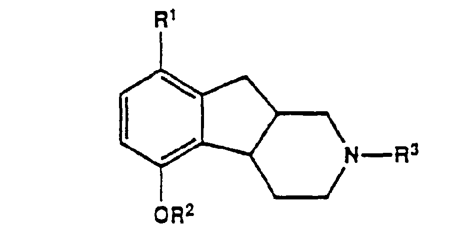 wherein R¹, R², and R³ are as defined therein

4. A process as defined by Claim 1 wherein R³ is selected from the group consisting of 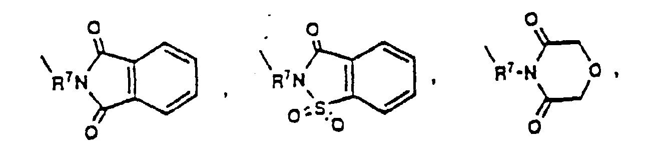 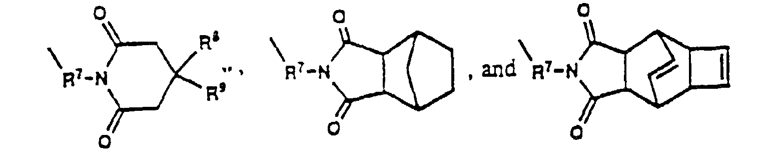 where R⁷, R⁸, and R⁹ are as defined therein.

5. A process as defined by Claim 2 for preparing a compound selected from the group consisting of
2R-(1,4-benzodioxanyl-2-methyl)-4-methoxy-1,2,3,3a,8,8a-hexahydroindeno[1,2-c]pyrrole;
2S-(1,4-benzodioxanyl-2-methyl)-4-methoxy-1,2,3,3a,8,8a-hexahydroindeno[1,2-c]pyrrole;
2-(3-(2,2-tetramethylene)glutarimidyl)propyl)-4-hydroxy-1,2,3,3a,8,8a-hexahydroindeno-[1,2-c]pyrrole;
2-(3-(3,3-tetramethylene)glutarimidyl)propyl)-4-methoxy-1,2,3,3a,8,8a-hexahydroindeno-[1,2-c]pyrrole;
2-(4-(3,3-tetramethylene)glutarimidyl)butyl-4-hydroxy-1,2,3,3a,8,8a-hexahydroindeno-[1,2-c]pyrrole;
2-(4-(3,3-dimethyl)glutarimidyl)butyl-4-methoxy-1,2,3,3a,8,8a-hexahydroindeno[1,2-c]pyrrole;
2-(4-(3,3-tetramethylene)glutarimidyl)butyl-4-methoxy-1,2,3,3a,8,8a-hexahydroindeno-[1,2-c]pyrrole;
2-(4-(3,3-tetramethylene)glutarimidyl)butyl-5-chloro-4-hydroxy-1,2,3,3a,8,8a-hexahydroindeno[1,2-c]pyrrole;
2-(4-(3,3-tetramethylene)glutarimidyl)butyl-5-chloro-4-methoxy-1,2,3,3a,8,8a-hexahydroindeno[1,2-c]pyrrole
2-(5-(3,3-tetramethylene)glutarimidyl)pentyl-4-methoxy-1,2,3,3a,8,8a-hexahydroindeno-[1,2-c]pyrrole;
2-(4-(3,3-tetramethylene)glutarimidyl)cis-buten-2-yl-4-methoxy-1,2,3,3a,8,8a-hexahydroindeno-[1,2-c]pyrrole
2-(4-(3,3-tetramethylene)glutarimidyl) trans-buten-2-yl-4-methoxy-1,2,3,3a,8,8a-hexahydroindeno-[1,2-c]pyrrole
4-hydroxy-2-(2-(4-Fluorobenzamido)ethyl)-1,2,3,3a,8,8a-hexahydroindeno[1,2-c]pyrrole;
4-methoxy-2-(2-(4-Fluorobenzamido)ethyl)-1,2,3,3a,8,8a-hexahydroindeno[1,2-c]pyrrole;
4-hydroxy-2-(4-(2-(1,2-Benzoisothiazolin-3-one-1,1-dioxide))butyl)-1,2,3,3a,8,8a-hexahydroindeno[1,2-c]-pyrrole;
4-methoxy-2-(4-(2-(1,2-Benzoisothiazolin-3-one-1,1-dioxide))butyl)-1,2,3, 3a,8,8a-hexahydroindeno[1,2-c]-pyrrole;
4-hydroxy-2-(4-(2-phthalimido)butyl)-1,2,3,3a,8,8a-hexahydroindeno-[1,2-c]pyrrole; and
4-methoxy-2-(4-(2-phthalimido)butyl)-1,2,3,3a,8,8a-hexahydroindezo-[1,2-c]pyrrole;and
pharmaceutically acceptable salts thereof.

6. A process as defined by Claim 3 for preparing a compound selected from the group consisting of
cis-2-(3,3-tetramethylene)glutarimidyl)propyl-5-methoxy-2,3,4,4a,9,9a-hexahydro-1H-indeno-[2,1-c]pyridine;
trans-2-(3,3-tetramethylene)glutarimidyl)propyl-5-methoxy-2,3,4,4a,9,9a-hexahydro-1H-indeno-[2,1-c]pyridine;
cis-2-(2,3-tetramethylene)glutarimidyl)butyl-5-methoxy-2,3,4,4a,9,9a-hexahydro-1H-indeno-[2,1-c]pyridine;
trans-2-(3,3-tetramethylene)glutarimidyl)butyl-5-methoxy-2,3,4,4a,9,9a-hexahydro-1H-indeno[2,1-c]pyridine;
trans-2(3-(2-(1,2-benzisothiazolin-3-one-1,1-dioxide))propyl)-5-methoxy-2,3,4,4a,9,9a-hexahydro-1H-indeno[2,1-c]pyridine;
trans-2(4-(2-(1,2-benzisothiazolin-3-one-1,1-dioxide))butyl)-5-methoxy-2,3,4,4a,9,9a-hexahydro-1H-indeno [2,2-c]pyridine, and
trans-2-(2-(4-fluorobenzamido)ethyl)-5-methoxy-2,3,4,4a,9,9a-hexahydro-1H-indeno-[2,1-c]pyridine hydrochloride; and
pharmaceutically acceptable salts thereof.

## Patentansprüche (Patentansprüche für folgende(n) Vertragsstaat(en) : AT, BE, CH, DE, FR, GB, IT, LI, LU, NL, SE)

1. Verbindung nach folgender Formel: 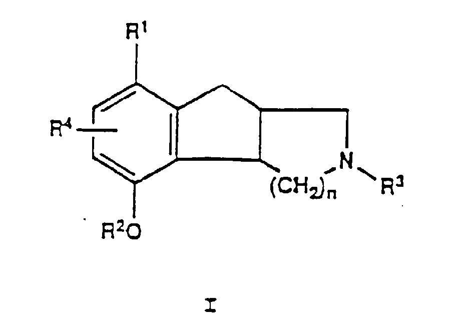
worin R¹ gleich Wasserstoff oder eine elektronenziehende Gruppe ist,
worin R² gleich Wasserstoff, Niederalkyl oder Arylalkyl ist,
worin n eine ganze Zahl mit einem Wert von 1 bis 3 ist,
worin R⁴ ein Glied ist, das aus der Gruppe gewählt ist, die aus folgendem besteht:
Wasserstoff,
Halogen,
Niederalkyl,
Niederalkoxy und
Aryl(niederalkyl), oder
worin R²0 und R⁴ zusammengenommen eine Alkylendioxybrücke ausbilden,
worin R³ ein Glied ist, das aus der Gruppe gewählt ist, die aus folgendem besteht:
Arylamidoalkenylen,
Arylamidoalkylen,
Aryl(niederalkyl)amidoalkenylen
Aryl(niederalkyl)amidoalkylen
Benzoalkylendioxyalkylen,
a) einer Gruppe nach folgender Formel: 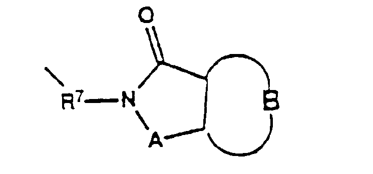
worin B gleich Benzo, Cyclohexyl oder ein Bicyclo-Ring ist,
worin A gleich CO, SO oder SO_{$2} ist, und
worin R⁷ ein divalenter aliphatischer
Kohlenwasserstoff ist,
b) einer Gruppe nach folgender Formel: 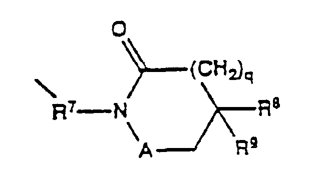 worin q eine ganze Zahl von 0 bis 3 ist, worin R⁸ und R⁹ gleich Wasserstoff oder Niederalkyl sind, oder worin R⁸ und R⁹ zusammengenommen einen Ring aus 5 bis 7 Giedern ausbilden, und worin A und R⁷ die oben definierte Bedeutung haben,
c) einer Gruppe nach folgender Formel: 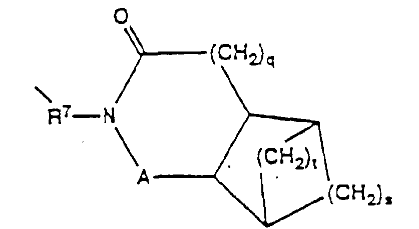 worin s und t voneinander unabhängige ganze
Zahlen von 1 bis 3 sind, und worin q, A und R⁷ die oben definierte Bedeutung haben, und
d) einer Gruppe nach folgender Formel: 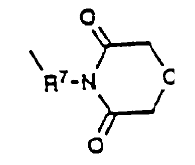
worin R⁷ die oben definierte Bedeutung hat,
worin Aryl mit mono- oder polycyclischen Kohlenwasserstoffen annelierte oder nicht-annelierte aromatische Ringsysteme bezeichnet, die eines oder mehrere Heteroatome in dem Ringsystem enthalten können, und
worin Aryl bei jedem Auftreten und die Ringsysteme in der R³ Gruppe wahlweise mit Halogen, Niederalkyl, mono- oder di-(niederalkyl)substituiertem Niederalkyl, (Niederalkyl)thio, halogensubstituiertem Niederalkyl, aminosubstituiertem Niederalkyl, mono- oder di(niederalkyl)-substicuiertem Amino, Niederalkenyl, Niederalkinyl,
Niederalkoxy, Aryloxy,
Aryl(niederalkyl), Hydroxy, Cyano, Amino, mono- und di(Niederalkyl)amino oder Nitro substituiert sein können, worin der Ausdruck "Niederalkyl" verzweigte oder unverzweigte gesättigte Kohlenwasserstoffradikale mit ein bis sechs Kohlenstoffatomen bezeichnet, und
die pharmazeutisch verträglichen Salze davon.

2. Verbindung nach Anspruch 1, die durch folgende Formel dargestellt wird: 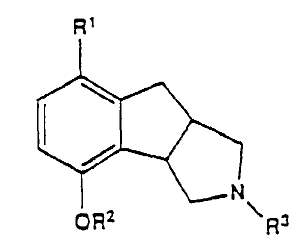 worin R¹, R² und R³ die hierin definierte Bedeutung haben.

3. Verbindung nach Anspruch 1, die durch die folgende Formel dargestellt wird: 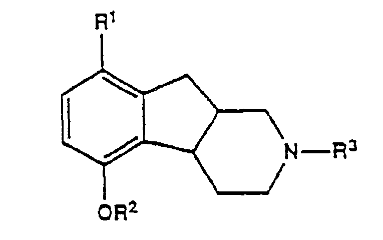 worin R¹, R² und R³ die hierin definierte Bedeutung haben.

4. Verbindung nach Anspruch 1, worin R³ aus der Gruppe gewählt ist, die aus folgendem besteht: 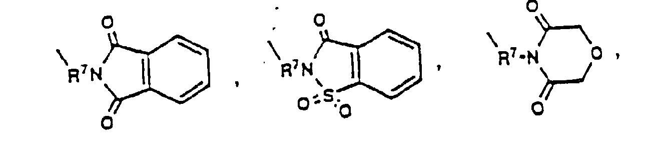 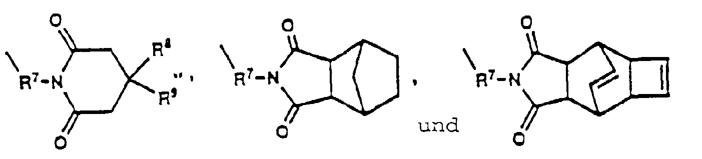 worin R⁷, R⁸ und R⁹ die hierin definierte Bedeutung haben.

5. Verbindung nach Anspruch 2, die aus der Gruppe gewählt ist, die aus folgendem besteht:
2R-(1,4-Benzodioxanyl-2-methyl)-4-methoxy-1,2,3,3a,8,8a-hexahydroindeno[1,2-c]pyrrol,
2S-(1,4-Benzodioxanyl-2-methyl)-4-methoxy-1,2,3,3a,8,8a-hexahydroindeno[1,2-c]pyrrol,
2-(3-(3,3-Tetramethylen)glutarimidyl)propyl-4-hydroxy-1,2,3,3a,8,8a-hexahydroindeno-[1,2-c]pyrrol,
2-(3-(3,3-Tetramethylen)glutarimidyl)propyl-4-methoxy-1,2,3,3a,8,8a-hexahydroindeno-[1,2-c]pyrrol,
2-(4-(3,3,-Tetramethylen)glutarimidyl)butyl-4-hydroxy-1,2,3,3a,8,8a-hexahydroindeno-[1,2-c]pyrrol,
2-(4-(3,3,-Dimethyl)glutarimidyl)butyl-4-methoxy-1,2,3,3a,8,8a-hexahydroindeno[1,2-c]pyrrol,
2-(4-(3,3,-Tetramethylen)glutarimidyl)butyl-4-methoxy-1,2,3,3a,8,8a-hexahydroindeno-[1,2-c]pyrrol,
2-(4-(3,3,-Tetramethylen)glutarimidyl)butyl-5-chlor-4-hydroxy-1,2,3,3a,8,8a-hexahydroindeno[1,2-c]pyrrol,
2-(4-(3,3-Tetramethylen)glutarimidyl)butyl-5-chlor-4-methoxy-1,2,3,3a,8,8a-hexahydroindeno[1,2-c]pyrrol,
2-(5-(3,3,-Tetramethylen)glutarimidyl)pentyl-4-methoxy-1,2,3,3a,8,8a-Hexahydroindeno-[1,2-c]pyrrol,
2-(4-(3,3-Tetramethylen)glutarimidyl)cis-buten-2-yl-4-methoxy-1,2,3,3a,8,8a-hexahydroindeno-[1,2-c]pyrrol,
2-(4-13,3,Tetramethylen)glutarimidyl)trans-buten-2-yl-4-methoxy-1,2,3,3a,8,8a-hexahydroindeno-[1,2-c]pyrrol,
4-Hydroxy-2-(2-(4-fluorbenzamido)ethyl)-1,2,3,3a,8,8a-hexahydroindeno[1,2-c]pyrrol,
4-Methoxy-2-(2-(4-fluorbenzamido)ethyl)-1,2,3,3a,8,8a-hexahydroindeno[1,2-c]pyrrol,
4-Hydroxy-2-(4-(2-(1,2-benzoisothiazolin-3-on-1,1-dioxid))butyl)-1,2,3,3a,8,8a-hexahydroindeno[1,2-c]-pyrrol,
4-Methoxy-2-(4-(2-(1,2-benzoisothiazolin-3-on-1,1-dioxid))butyl)-1,2,3,3a,8,8a-hexahydroindezo[1,2-c]-pyrrol,
4-Hydroxy-2-(4-(2-phthalimido)butyl)-1,2,3,3a,8,8a-hexahydroindeno-[1,2-c]pyrrol, und
4-Methoxy-2-(4-(2-Phthalimido)butyl)-1,2,3,3a,8,8a-Hexahydroindeno-[1,2-c]pyrrol,und
pharmazeutisch verträgliche Salze davon.

6. Verbindung nach Anspruch 3, die aus der Gruppe gewählt ist, die aus folgendem besteht:
cis-2-(3,3-Tetramethylen)glutarimidyl)propyl-5-methoxy-2,3,4,4a,9,9a-hexahydro-1H-indeno-[2,1-c]pyridin,
trans-2-(3,3-Tetramethylen)glutarimidyl)propyl-5-methoxy-2,3,4,4a,9,9a-hexahydro-1 H-indeno-[2,1-c]pyridin,
cis-2-(3,3-Tetramethylen)glutarimidyl)butyl-5-methoxy-2,3,4,4a,9,9a-hexahydro-1H-indeno-[2,1-c]pyridin,
trans-2-(3,3-Tetramethylen)glutarimidyl)butyl-5-methoxy-2,3,4,4a,9,9a-hexahydro-IH-indeno [2,1-c]pyridin,
trans-2-(3-(2-(1,2-Benzisothiazolin-3-on-1,1-dioxid))propyl)-5-methoxy-2,3,4,4a,9,9a-hexahydro-IH-indeno [2,1-c]pyridin,
trans-2(4-(2-(1,2-Benzisothiazolin-3-on-1,1-dioxid))butyl)-5-methoxy-2,3,4,4a,9,9a-hexahydro-1H-indeno [2,1-c]pyridin, und
trans-2-(2-(4-Fluorbenzamido)ethyl)-5-methoxy-2,3,4,4a,9,9a-hexahydro-1 H-indeno-[2,1-c]pyridinhydrochlorid, und
pharmazeutisch verträgliche Salze davon.

7. Verbindung nach Anspruch 1 zur Verwendung als Medikament.

8. Pharmazeutische Zusammensetzung, die eine Verbindung nach Anspruch 1 oder ein pharmazeutisch verträgliches Salz davon in Kombination mit einem pharmazeutisch verträglichen Träger umfaßt.

9. Verwendung einer Verbindung nach Anspruch 1 zur Herstellung eines Medikamentes zur Beeinflussung der zentralen 5-HT,_{A}- Rezeptoren bei einem Säugerpatienten, der einer solchen Behandlung bedarf.

10. Verwendung einer Verbindung nach Anspruch 1 zur Herstellung eines Medikamentes zur Behandlung von Hochdruck, Angstzuständen oder Depression bei einem Säugerpatienten der einer solchen Behandlung bedarf.

## Patentansprüche (Patentansprüche für folgende(n) Vertragsstaat(en) : ES)

1. Verfahren zur Herstellung einer Verbindung nach folgender Formel: 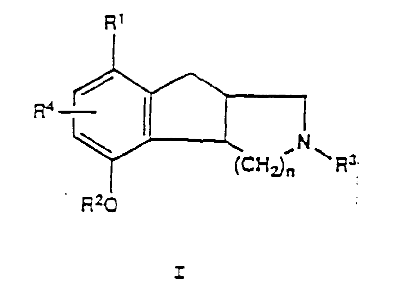
worin R¹ gleich Wasserstoff oder eine elektronenziehende Gruppe ist,
worin R² gleich Wasserstoff, Niederalkyl oder Arylalkyl ist,
worin n eine ganze Zahl mit einem Wert von 1 bis 3 ist,
worin R⁴ ein Glied ist, das aus der Gruppe gewählt ist, die aus folgendem besteht:
Wasserstoff,
Halogen,
Niederalkyl,
Niederalkoxy und
Aryl(niederalkyl), oder
worin R²0 und R⁴ zusammengenommen eine Alkylendioxybrücke ausbilden,
worin R³ ein Glied ist, das aus der Gruppe gewählt ist, die aus folgendem besteht:
Arylamidoalkenylen,
Arylamidoalkylen,
Aryl(niederalkyl)amidoalkenylen,
Aryl(niederalkyl)amidoalkylen,
Benzoalkylendioxcyalkylen,
a) einer Gruppe nach folgender Formel: 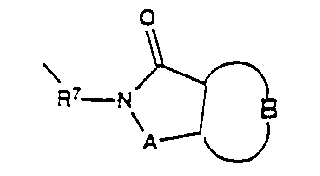
worin B gleich Benzo, Cyclohexyl oder ein Bicyclo-Ring ist,
worin A gleich CO, SO oder S0₂ ist, und
worin R⁷ ein divalenter aliphatischer Kohleniwasserstoff ist,
b) einer Gruppe nach folgender Formel: 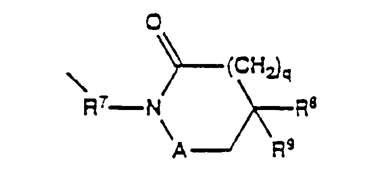 worin q eine ganze Zahl von 0 bis 3 ist, worin
R⁸ und R⁹ gleich Wasserstoff oder Niederalkyl sind, oder worin R⁸ und R⁹ zusammengenommen einen
Ring aus 5 bis 7 Giedern ausbilden, und worin A und R⁷ die oben definierte Bedeutung haben,
c) einer Gruppe nach folgender Formel : 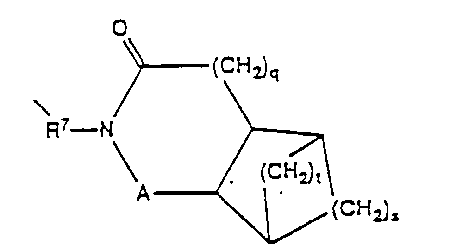 worin s und t voneinander unabhängige ganze Zahlen von 1 bis 3 sind, und worin q, A und R⁷ die oben definierte Bedeutung haben, und
d) einer Gruppe nach folgender Formel: 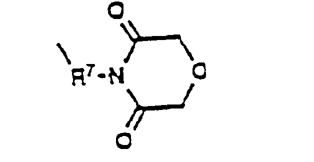
worin R⁷ die oben definierte Bedeutung hat, worin Aryl mit mono- oder polycyclischen Kohlenwasserstoffen annelierte oder nicht-annelierte aromatische Ringsysteme bezeichnet, die eines oder mehrere Heteroatome in dem Ringsystem enthalten können, und
worin Aryl bei jedem Auftreten und die Ringsysteme in der R³ Gruppe wahlweise mit Halogen, Niederalkyl, mono- oder di-(niederalkyl)substituiertem Niederalkyl, (Niederalkyl)thio, halogensubstituiertem Niederalkyl, aminosubstituiertem Niederalkyl, mono- oder di(niederalkyl)-substituiertem Almino, Niederalkenyl, Niederalkinyl,
Niederalkoxy, Aryloxy,
Aryl(niederalkyl), Hydroxy, Cyano, Amino, mono- und di(Niederalkyl)amino oder Nitro substituiert sein können, und worin der Ausdruck "Niederalkyl" verzweigte oder unverzweigte gesättigte Kohlenwasserstoffradikale mit ein bis sechs Kohlenstoffatomen bezeichnet, und
die pharmazeutisch verträglichen Salze davon, wobei das Verfahren die Umsetzung einer Verbindung nach folgender Formel: 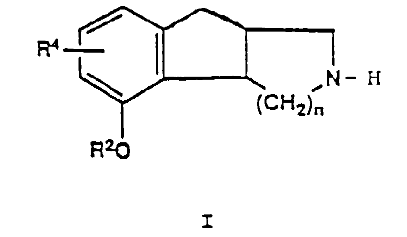 ,worin R¹, R², R⁴ und n die oben definierte Bedeutung haben, mit einer Verbindung der Formel R³⁻Halogen umfaßt.

2. Verfahren nach Anspruch 1 zur Herstellung einer Verbindung, die durch folgende Formel dargestellt ist: 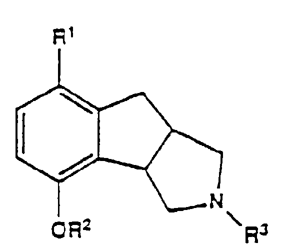 ,worin R¹, R² und R³ die hierin definierte Bedeutung haben.

3. Verfahren nach Anspruch 1 zur Herstellung einer Verbindung, die durch folgende Formel dargestellt wird: 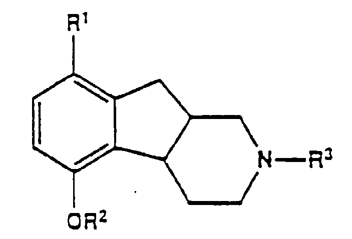 worin R¹, R² und R³ die hierin definierte Bedeutung haben.

4. Verfahen nach Anspruch 1, worin R³ aus der Gruppe gewählt ist, die aus folgendem besteht: 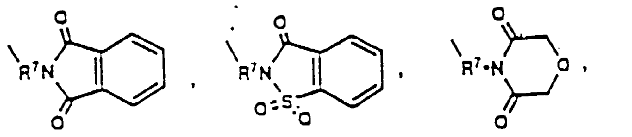 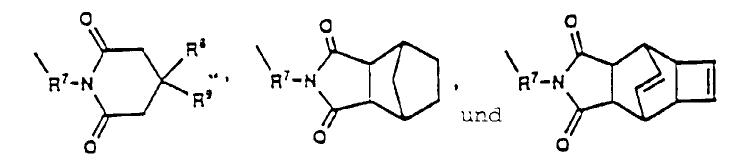 ,worin R⁷, R⁸ und R⁹ die hierin definierte Bedeutung haben.

5. Verfahren nach Anspruch 2 zur Herstellung einer Verbindung, die aus der Gruppe gewählt ist die aus folgendem besteht:
2R-(1,4-Benzodioxanyl-2-methyl)-4-methoxy-1,2,3,3a,8,8a-hexahydroindeno[1,2-c]pyrrol,
2S-(1,4-Benzodioxanyl-2-methyl)-4-methoxy-1,2,3,3a,8,8a-hexahydroindeno[1,2-c]pyrrol,
2-(3-(3,3-Tetramethylen)glutarimidyl)propyl-4-hydroxo-1,2,3,3a,8,8a-hexahydroindeno-[1,2-c]pyrrol,
2-(3-(3,3-Tetramethylen)glutarimidyl)propyl-4-methoxy-1,2,3,3a,8,8a-hexahydroindeno-[1,2-c]pyrrol,
2-(4-(3,3,-Tetramethylen)glutarimidyl)butyl-4-hydroxy-1,2,3,3a,8,8a-hexahydroindeno-[1,2-c]pyrrol,
2-(4-(3,3,-Dimethyl)glutarimidyl)butyl-4-methoxy-1,2,3,3a,8,8a-hexahydroindeno[1,2-c]pyrrol,
2-(4-(3,3,-Tetramethylen)glutarimidyl)butyl-4-methoxy-1,2,3,3a,8,8a-hexahydroindeno-[1,2-c]pyrrol,
2-(4-(3,3,-Tetramethylen)glutarimidyl)butyl-5-chlor-4-hydroxy-1,2,3,3a,8,8a-hexahydro-indeno[1,2-c]pyrrol,
2-(4-(3,3-Tetramethylen)glutarimidyl)butyl-5-chlor-4-methoxy-1,2,3,3a,8,8a-hexahydroindeno[1,2-c]pyrrol,
2-(5-(3,3,-Tetramethylen)glutarimidyl)pentyl-4-methoxy-1,2,3,3a,8,8a-Hexahydroindeno-[1,2-c]pyrrol,
2-(4-(3,3-Tetramethylen)glutarimidyl)cis-buten-2-yl-4-methoxy-1,2,3,3a,8,8a-hexahydroindeno-[1,2-c]pyrrol,
2-(4-(3,3,Tetramethylen)glutarimidyl)trans-buten-2-yl-4-methoxy-1,2,3,3a,8,8a-hexahydroindeno-[1,2-c]pyrrol,
4-Hydroxy-2-(2-(4-fluorbenzamido)ethyl)-1,2,3,3a,8,8a-hexahydroindeno[1,2-c]pyrrol,
4-Methoxy-2-(2-(4-fluorbenzamido)ethyl)-1,2,3,3a,8,8a-hexahydroindeno[1,2-c]pyrrol,
4-Hydroxy-2-(4-(2-(1,2-benzoisothiazolin-3-on-1,1-dioxid))butyl)-1,2, 3,3a,8,8a-hexahydroindeno[1,2-c]-pyrrol,
4-Methoxy-2-(4-(2-(1,2-benzoisothiazolin-3-on-1,1 -dioxid))butyl)-1,2,3,3a,8,8a-hexahydroindeno[1,2-c]-pyrrol,
4-Hydroxy-2-(4-(2-phthalimido)butyl)-1,2,3,3a,8,8a-hexahydroindeno-[1,2-c]pyrrol, und
4-Methoxy-2-(4-(2-Phthalimido)butyl)-1,2,3,3a,8,8a-Hexahydroindeno-[1,2-c]pyrrol,und von
pharmazeutisch verträglichen Salzen davon.

6. Verfahren nach Anspruch 3 zur Herstellung einer Verbindung, die aus der Gruppe gewählt ist, die aus folgendem besteht:
cis-2-(3,3-Tetramethylen)glutarimidy)propyl-5-methoxy-2,3,4,4a,9,9a-hexahydro-1H-indeno-[2,1-c]pyridin,
trans-2-(3,3-Tetramethylen)glutarimidyl)propyl-5-methoxy-2,3,4,4a,9,9a-hexahydro-1 H-indeno-[2,1-c]pyridin,
cis-2-(3,3-Tetramethylen)glutarimidyl)butyl-5-methoxy-2,3,4,4a,9,9a-hexahydro-1H-indeno-[2,1-c]pyridin,
trans-2-(3,3-Tetramethylen)glutarimidyl)butyl-5-methoxy-2,3,4,4a,9,9a-hexahydro-1 H-indeno [2,1-c]pyridin,
trans-2-(3-(2-(1,2-Benzisothiazolin-3-on-1,1-dioxid))propyl)-5-methoxy-2,3,4,4a,9,9a-hexahydro-1H-indeno [2,1-c]pyridin,
trans-2(4-(2-(1,2-Benzisothiazolin-3-on-1,1-dioxid))butyl)-5-methoxy-2,3,4,4a,9,9a-hexahydro-1H-indeno [2,1-c]pyridin, und
trans-2-(2-(4-Fluorbenzamido)ethyl)-5-methoxy-2,3,4,4a,9,9a-hexahydro-1 H-indeno-[2,1-c]pyridinhydrochlorid, und
pharmazeutisch verträglichen Salzen davon.

## Revendications (Revendications pour l'(les) Etat(s) contractant(s) suivant(s) : AT, BE, CH, DE, FR, GB, IT, LI, LU, NL, SE)

1. Composé de formule 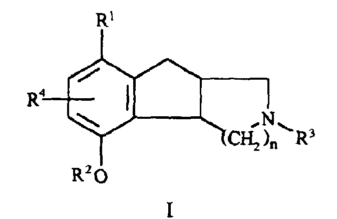 dans laquelle
R¹ est un atome d'hydrogène ou un groupe attracteur d'électrons;
R² est un atome d'hydrogène ou un groupe alkyle inférieur ou arylalkyle;
n est un entier d'une valeur de 1 à 3;
R⁴ est un membre choisi dans le groupe constitué par
un atome d'hydrogène,
un atome d'halogène,
un groupe alkyle inférieur,
un groupe alcoxy inférieur et
un groupe aryl(alkyle inférieur); ou
R²0 et R⁴ forment ensemble un pont alkylènedioxy;
R³ est un membre choisi dans le groupe constitué par
un groupe arylamidoalcénylène,
un groupe arylamidoalkylène,
un groupe aryl(alkyl inférieur)amidoalcénylène,
un groupe aryl(alkyl inférieur)amidoalkylène,
un groupe benzoalkylènedioxyalkylène,
a) un groupe de formule 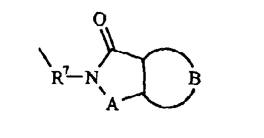
dans laquelle B est un groupe benzo, cyclohexyle ou un noyau bicyclo:
A est un groupe CO, SO ou S0₂, et
R⁷ est un hydrocarbure aliphatique divalent;
b) un groupe de formule 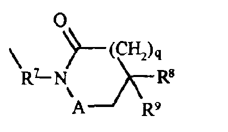
dans laquelle q est un entier de 0 à 3,
R⁸ et R⁹ sont des atomes d'hydrogène ou des groupes alkyle inférieur, ou
R⁸ et R⁹ forment ensemble un cycle de 5 à 7 chaînons, et
A et R⁷ ont la définition indiquée ci-dessus;
c) un groupe de formule 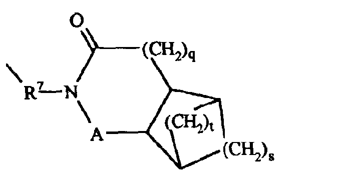 dans laquelle s et t sont des entiers indépendants de 1 à 3, et q, A et R⁷ ont la définition donnée ci-dessus; et
d) un groupe de formule 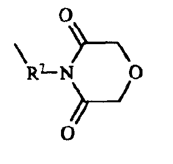
dans laquelle R⁷ a la définition donnée ci-dessus;
où les groupes aryle désignent des systèmes cycliques aromatiques hydrocarbonés mono- ou polycycli-
ques, condensés ou non condensés, qui peuvent contenir un ou plusieurs hétéroatomes dans le système cyclique; et
les groupes aryle, partout où ils apparaissent, et les systèmes cycliques dans le groupe R³ peuvent éventuellement être substitués par halogène, alkyle inférieur, alkyle inférieur substitué par un ou deux groupes alkyle inférieur, (alkyl inférieur)thio, halogénoalkyle inférieur, aminoalkyle inférieur, amino substitué par un ou deux groupes alkyle inférieur, alcényle inférieur, alcynyle inférieur, alcoxy inférieur, aryloxy, aryl (alkyle inférieur), hydroxy, cyano, amino, mono et di(alkyl inférieur)amino, ou nitro; et
leurs sels d'acides pharmaceutiquement acceptables, l'expression "alkyle inférieur" désignant des radicaux hydrocarbonés saturés linéaires ou ramifiés ayant de 1 à 6 atomes de carbone.

2. Composé selon la revendication 1, qui est représenté par la formule 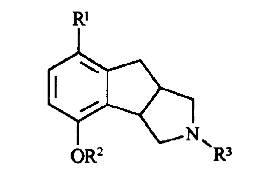 dans laquelle R¹, R² et R³ ont la définition donnée ci-dessus.

3. Composé selon la revendication 1, représenté par la formule 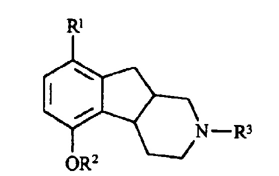 dans laquelle R¹, R² et R³ ont la définition donnée ci-dessus.

4. Composé selon la revendication 1, dans lequel R³ est choisi dans le groupe constitué par 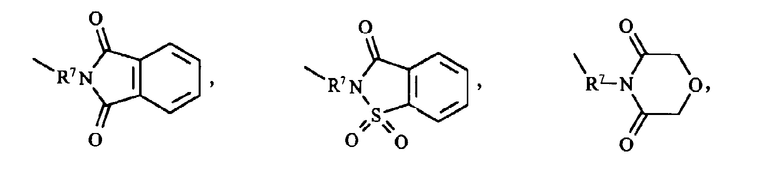 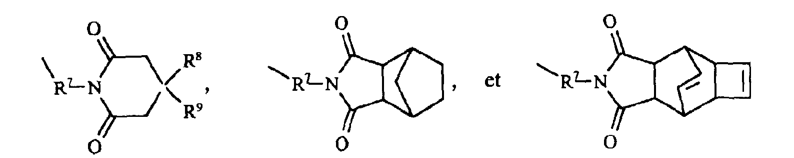 où R⁷, R⁸ et R⁹ ont la définition donnée ci-dessus.

5. Composé selon la revendication 2, choisi dans le groupe constitué par
le 2R-(1,4-benzodioxanyl-2-méthyl)-4-méthoxy-1,2,3,3a,8,8a-hexahydroindeno[1,2-c]pyrrole;
le 2S-(1,4-benzodioxanyl-2-méthyl)-4-méthoxy-1,2,3,3a,8,8a-hexahydroindéno[1,2-c]pyrrole;
le 2-(3-(3,3-tétraméthylène)glutarimidyl)propyl)-4-hydroxy-1,2,3,3a,8,8a-hexahydroindéno[1,2-c]pyrrole;
le 2-(3-(3,3-tétraméthylène)glutarimidyl)propyl)-4-méthoxy-1,2,3,3a,8,8a-hexahydroindéno[1,2-c]pyrrole;
le 2-(4-(3,3-tétraméthylène)glutrimidyl)butyl)-4-hydroxy-1,2,3,3a,8,8a-hexahydroindéno[1,2-c]pyrrole;
le 2-(4-(3,3-diméthyl)glutarimidyl)butyl)-4-méthoxy-1,2,3,3a,8,8a-hexahydroindéno[1,2-c]pyrrole;
le 2-(4-(3,3-tétraméthylène)glutarimidyl)butyl)-4-méthoxy-1,2,3,3a,8,8a-hexahydroindéno[1,2-c]pyrrole;
le 2-(4-(3,3-tétraméthylène)glutarimidyl)butyl)-5-chloro-4-hydroxy-1,2,3,3a, 8,8a-hexahydroindéno[1,2-c]pyrrole;
le 2-(4-(3,3-tétraméthylène)glutarimidyl)butyl)-5-chloro-4-méthoxy-1,2,3,3a,8,8a-hexahydroindéno[1,2-c] pyrrole;
le 2-(5-(3,3-tétraméthylène)glutarimidyl)pentyl)-4-méthoxy-1,2,3,3a,8,8a-hexahydroindéno[1,2-c]pyrrole;
le 2-(4-(3,3-tétraméthyléne)glutarimidyl)cis-butène-2-yl-4-méthoxy-1,2,3,3a,8, 8a-hexahydroindéno[1,2-c] pyrrole;
le 2-(4-(3,3-tétraméthylène)glutarimidyl)trans-butène-2-yl-4-méthoxy-1,2,3,3a,8,8a-hexahydroindéno[1,2-c] pyrrole;
le 4-hydroxy-2-(2-(4-fluorobenzamido)éthyl)-1,2,3,3a,8,8a-hexahydroindéno-[1,2-c]pyrrole;
le 4-méthoxy-2-(2-(4-fluorobenzamido)éthyl)-1,2,3,3a,8,8a-hexahydroindéno-[1,2-c]pyrrole;
le 4-hydroxy-2-(4-(2-(1,2-benzisothiazoline-3-one-1, -dioxyde))butyl)-1,2,3, 3a,8,8a-hexahydroindéno[1,2-c] pyrrole
le 4-méthoxy-2-(4-(2-(1,2-benzisothiazoline-3-one-1,1-dioxyde))butyl)-1,2,3, 3a,8,8a-hexahydroindéno [1,2-c]pyrrole;
le 4-hydroxy-2-(4-(2-phtalimido)butyl)-1,2,3,3a,8,8a-hexahydroindéno[1,2-c]pyrrole; et
le 4-méthoxy-2-(4-(2-phtalimido)butyl)-1,2,3,3a,8,8a-hexahydroindéno[1,2-c]pyrrole;et
leurs sels pharmaceutiquement acceptables.

6. Composé selon la revendication 3, choisi dans le groupe constitué par
la cis-2-(3,3-tétraméthylène)glutarimidyl)propyl-5-méthoxy-2,3,4,4a,9,9a-hexahydro-1H-indéno[2,1-c]pyridine;
la trans-2-(3,3-tétraméthylène)glutarimidyl)propyl-5-méthoxy-2,3,4,4a,9,9a-hexahydro-1H-indéno[2,1-c]pyridine;
la cis-2-(3,3-tétraméthylène)glutarimidyl)butyl-5-méthoxy-2,3,4,4a,9,9a-hexahydro-1H-indéno[2,1-c]pyridine;
la trans-2-(3,3-tétraméthylène)glutarimidyl)butyl-5-méthoxy-2,3,4,4a,9,9a-hexahydro-1H-indénos[2,1-c]pyridine;
la trans-2-(3-(2-(1,2-benzisothiazoline-3-one-1,1-dioxyde))propyl)-5-méthoxy-2,3,4,4a,9,9a-hexahydro-1H-indéno[2,1-c]pyridine;
la trans-2-(4-(2-(1,2-benzisothiazoline-3-one-1,1-dioxyde))butyl)-5-méthoxy-2,3,4,4a,9,9a-hexahydro-1H-in- déno[2,1-c]pyridine; et
le chlorhydrate de trans-2-(2-(4-fluorobenzamido)éthyl)-5-méthoxy-2,3,4,4a,9,9a-hexahydro-1H-indéno [2,1-c]pyridine; et
leurs sels pharmaceutiquement acceptables.

7. Composé selon la revendication 1 à utiliser comme médicament.

8. Composition pharmaceutique comprenant un composé selon la revendication 1 ou un de ses sels pharmaceutiquement acceptables, en combinaison avec un support pharmaceutiquement acceptable.

9. Utilisation d'un composé selon la revendication 1 pour la fabrication d'un médicament destiné à la modulation des récepteurs centraux de la 5-HT_{1A} chez un mammifère ayant besoin d'un tel traitement.

10. Utilisation d'un composé selon la revendication 1 pour la fabrication d'un médicament pour le traitement de l'hypertension, de l'anxiété ou de la dépression chez un mammifère ayant besoin d'un tel traitement.

## Revendications (Revendications pour l'(les) Etat(s) contractant(s) suivant(s) : ES)

1. Procédé de préparation d'un composé de formule 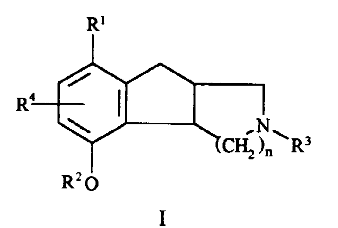 dans laquelle
R¹ est un atome d'hydrogène ou un groupe attracteur d'électrons;
R² est un atome d'hydrogène ou un groupe alkyle inférieur ou arylalkyle;
n est un entier d'une valeur de 1 à 3;
R⁴ est un membre choisi dans le groupe constitué par
un atome d'hydrogène,
un atome d'halogène,
un groupe alkyle inférieur,
un groupe alcoxy inférieur et
un groupe aryl(alkyle inférieur); ou
R²0 et R⁴ forment ensemble un pont alkylènedioxy;
R³ est un membre choisi dans le groupe constitué par
un groupe arylamidoalcénylène,
un groupe arylamidoalkylène,
un groupe aryl(alkyl inférieur)amidoalcénylène,
un groupe aryl(alkyl inférieur)amidoalkylène,
un groupe benzoalkylènedioxyalkylène,
a) un groupe de formule 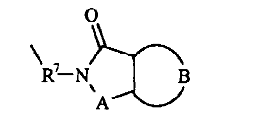
dans laquelle B est un groupe benzo, cyclohexyle ou un noyau bicyclo;
A est un groupe CO, SO ou S0₂ et
R⁷ est un hydrocarbure aliphatique divalent;
b) un groupe de formule 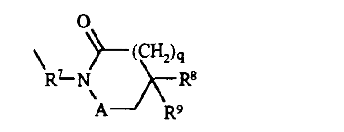
dans laquelle q est un entier de 0 à 3,
R⁸ et R⁹ sont des atomes d'hydrogène ou des groupes alkyle inférieur, ou
R⁸ et R⁹ forment ensemble un cycle de 5 à 7 chaînons, et
A et R⁷ ont la définition indiquée ci-dessus;
c) un groupe de formule 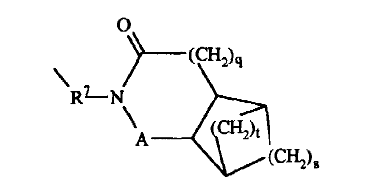 dans laquelle s et t sont des entiers indépendants de 1 à 3, et q, A et R⁷ ont la définition donnée ci-dessus; et
d) un groupe de formule 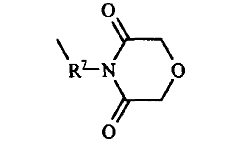
dans laquelle R⁷ a la définition donnée ci-dessus;
où les groupes aryle désignent des systèmes cycliques aromatiques hydrocarbonés mono- ou polycycliques, condensés ou non condensés, qui peuvent contenir un ou plusieurs hétéroatomes dans le système cyclique; et
les groupes aryle, partout où ils apparaissent, et les systèmes cycliques dans le groupe R³ peuvent éventuellement être substitués par halogène, alkyle inférieur, alkyle inférieur substitué par un ou deux groupes alkyle inférieur, (alkyl inférieur)thio, halogénoalkyle inférieur, aminoalkyle inférieur, amino substitué par un ou deux groupes alkyle inférieur, alcényle inférieur, alcynyle inférieur, alcoxy inférieur, aryloxy, aryl (alkyle inférieur), hydroxy, cyano, amino, mono et di(alkyl inférieur)amino, ou nitro; et
l'expression "alkyle inférieur" désigne des radicaux hydrocarbonés saturés linéaires ou ramifiés ayant de 1 à 6 atomes de carbone; et
de leurs sels d'acides pharmaceutiquement acceptables, ledit procédé comprenant la réaction d'un composé de formule 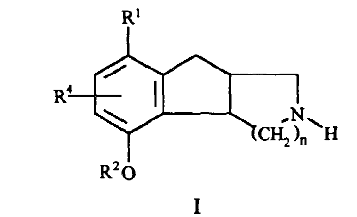 dans laquelle R¹, R², R⁴ et n ont la définition donnée ci-dessus, avec un composé de formule R³⁻halogène.

2. Procédé selon la revendication 1, pour la préparation d'un composé représenté par la formule 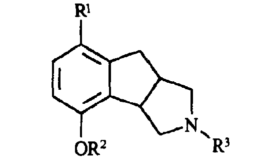 dans laquelle R¹, R² et R³ ont la signification définie ci-dessus.

3. Procédé selon la revendication 1, pour la préparation d'un composé représenté par la formule 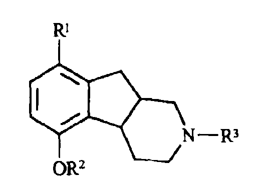 dans laquelle R¹, R² et R³ ont la signification définie ci-dessus.

4. Procédé selon la revendication 1, dans lequel R³ est choisi dans le groupe constitué par 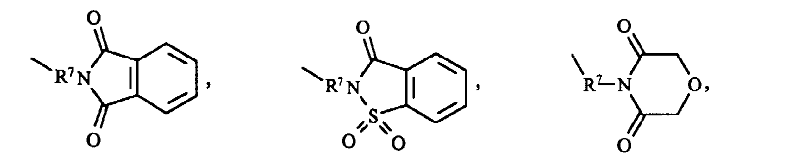 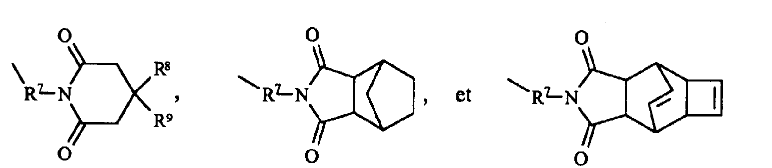 où R⁷, R⁸ et R⁹ ont la définition donnée ci-dessus.

5. Procédé selon la revendication 2, pour la préparation d'un composé choisi dans le groupe constitué par
le 2R-(1,4-benzodioxanyl-2-méthyl)-4-méthoxy-1,2,3,3a,8,8a-hexahydroindéno[1,2-c]pyrrole;
le 2S-(1,4-benzodioxanyl-2-méthyl)-4-méthoxy-1,2,3,3a,8,8a-hexahydroindéno[1,2-c]pyrrole;
le 2-(3-(3,3-tétraméthylène)glutarimidyl)propyl)-4-hydroxy-1,2,3,3a,8,8a-hexahydroindéno[1,2-c]pyrrole;
le 2-(3-(3,3-tétraméthylène)glutarimidyl)propyl)-4-méthoxy-1,2,3,3a,8,8a-hexahydroindéno[1,2-c]pyrrole;
le 2-(4-(3,3-tétraméthylène)glutarimidyl)butyl)-4-hydroxy-1,2,3,3a,8,8a-hexahydroindéno[1,2-c]pyrrole;
le 2-(4-(3,3-diméthyl)glutarimidyl)butyl)-4-méthoxy-1,2,3,3a,8,8a-hexahydroindéno[1,2-c]pyrrole;
le 2-(4-(3,3-tétraméthylène)glutarimidyl)butyl)-4-méthoxy-1,2,3,3a,8,8a-hexahydroindéno[1,2-c]pyrrole;
le 2-(4-(3,3-tétraméthylène)glutarimidyl)butyl)-5-chloro-4-hydroxy-1,2,3,3a,8,8a-hexahydroindéno[1,2-c]pyrrole;
le 2-(4-(3,3-tétraméthylène)glutarimidyl)butyl)-5-chloro-4-méthoxy-1,2,3,3a,8,8a-hexahydroindëno[1,2-c] pyrrole;
le 2-(5-(3,3-tétraméthylène)glutarimidyl)pentyl)-4-méthoxy-1,2,3,3a,8,8a-hexahydroindéno[1,2-c]pyrrole;
le 2-(4-(3,3-tétraméthylène)glutarimidyl)cis-butène-2-yl-4-méthoxy-1,2,3,3a,8,8a-hexahydroindéno[1,2-c] pyrrole;
le 2-(4-(3,3-tétraméthylène)glutarimidyl)trans-butène-2-yl-4-méthoxy-1,2,3,3a,8,8a-hexahydroindéno[1,2-c] pyrrole;
le 4-hydroxy-2-(2-(4-fluorobenzamido)éthyl)-1,2,3,3a,8,8a-hexahydroindéno-[1,2-c]pyrrole;
le 4-méthoxy-2-(2-(4-fluorobenzamido)éthyl)-1,2,3,3a,8,8a-hexahydroindéno-[1,2-c]pyrrole;
le 4-hydroxy-2-(4-(2-(1,2-benzisothiazoline-3-one-1, -dioxyde))butyl)-1,2,3, 3a,8,8a-hexahydroindéno[1,2-c] pyrrole;
le 4-méthoxy-2-(4-(2-(1,2-benzisothiazoline-3-one-1,1-dioxyde))butyl)-1,2,3,3a,8,8a-hexahydroindéno [1,2-c]pyrrole;
le 4-hydroxy-2-(4-(2-phtalimido)butyl)-1,2,3,3a,8,8a-hexahydroindéno[1,2-c]pyrrole;et
le 4-méthoxy-2-(4-(2-phtalimido)butyl)-1,2,3,3a,8,8a-hexahydroindéno[1,2-c]pyrrole;et
leurs sels pharmaceutiquement acceptables.

6. Procédé selon la revendication 3, pour la préparation d'un composé choisi dans le groupe constitué par
la cis-2-(3,3-tétraméthylène)glutarimidyl)propyl-5-méthoxy-2,3,4,4a,9,9a-hexahydro-1H-indéno[2,1-c]pyridine;
la trans-2-(3,3-tétraméthylène)glutarimidyl)propyl-5-méthoxy-2,3,4,4a,9,9a-hexahydro-1H-indéno[2,1-c]pyridine;
la cis-2-(3,3-tétraméthylène)glutarimidyl)butyl-5-méthoxy-2,3,4,4a,9,9a-hexahydro-1H-indéno[2,1-c]pyridine;
la trans-2-(3,3-tétraméthylène)glutarimidyl)butyl-5-méthoxy-2,3,4,4a,9,9a-hexahydro-1H-indéno[2,1-c]pyridine;
la trans-2-(3-(2-(1,2-benzisothiazoline-3-one-1,1-dioxyde))propyl)-5-méthoxy-2,3,4,4a,9,9a-hexahydro- 1H-indéno[2,1-c]pyridine;
la trans-2-(4-(2-(1,2-benzisothiazoline-3-one-1,1-dioxyde))butyl)-5-méthoxy-2,3,4,4a,9,9a-hexahydro-1H-in- déno[2,1-c]pyridine; et
le chlorhydrate de trans-2-(2-(4-fluorobenzamido)éthyl)-5-méthoxy-2,3,4,4a,9,9a-hexahydro-1H-indéno [2,1-c]pyridine; et
leurs sels pharmaceutiquement acceptables.
